# EUROPEAN PATENT SPECIFICATION

(11) **EP 4 165 039 B1**
(45) Date of publication and mention of the grant of the patent: **21.08.2024**
(21) Application number: 20945703.5
(22) Date of filing: 16.07.2020
(51) Int. Cl.: C07D 417/14, A61K 31/427, A61P 31/04, A61K 31/433

(54) **NOVEL MONOBACTAM COMPOUNDS, THEIR PREPARATION AND USE AS ANTIBACTERIAL AGENTS**
MONOBACTAM-VERBINDUNGEN, IHRE HERSTELLUNG UND IHRE VERWENDUNG ALS ANTIBAKTERIELLE MITTEL
NOUVEAUX COMPOSÉS MONOBACTAM, LEUR PRÉPARATION ET LEUR UTILISATION EN TANT QU'AGENTS ANTIBACTÉRIENS

(43) Date of publication of application: 19.04.2023
(73) Proprietor: Ningxia Academy of Agriculture and Forestry Sciences, Yinchuan, Ningxia 750002 (CN)
(72) Inventor: YANG, Zhixiang, Yinchuan, Ningxia 750002 (CN); YANG, Haikang, Yinchuan, Ningxia 750002 (CN); JI, Jinbo, Yinchuan, Ningxia 750002 (CN); ZHAI, Lijuan, Yinchuan, Ningxia 750002 (CN); HE, Lili, Yinchuan, Ningxia 750002 (CN); SUN, Jian, Yinchuan, Ningxia 750002 (CN); MYO, Koko, Yinchuan, Ningxia 750002 (CN); TANG, Dong, Yinchuan, Ningxia 750002 (CN); GAO, Yuanyu, Yinchuan, Ningxia 750002 (CN); LIU, Yuanbai, Yinchuan, Ningxia 750002 (CN); MU, Yangxiu, Yinchuan, Ningxia 750002 (CN); JI, Jingwen, Yinchuan, Ningxia 750002 (CN); IQBAL, Zafar, Yinchuan, Ningxia 750002 (CN); THU, Zawmin, Yinchuan, Ningxia 750002 (CN); JIANG, Rui, Yinchuan, Ningxia 750002 (CN); MA, Xueqin, Yinchuan, Ningxia 750004 (CN); YU, Jianqiang, Yinchuan, Ningxia 750004 (CN)
(74) Representative: Bayramoglu et al.
(86) International application number: PCT/CN2020/102265
(87) International publication number: WO 2022/011626

(56) References cited:
- WO-A1-2013/110643
- WO-A1-2019/020810
- CN-A- 104 203 237
- CN-A- 106 164 072
- CN-A- 110 022 857
- CN-A- 110 312 715
- FOLKERT RECK, ALUN BERMINGHAM, JOHANNE BLAIS, VLADIMIR CAPKA, TARYN CARIAGA, ANTHONY CASAREZ, RICHARD COLVIN, CHARLES R. DEAN, ALE: "Optimization of novel monobactams with activity against carbapenem-resistant Enterobacteriaceae – Identification of LYS228", BIOORGANIC & MEDICINAL CHEMISTRY LETTERS, ELSEVIER, AMSTERDAM, NL, vol. 28, no. 4, 1 February 2018 (2018-02-01), AMSTERDAM, NL , pages 748 - 755, XP055535945, ISSN: 0960-894X, DOI: 10.1016/j.bmcl.2018.01.006
- RUGGIU FIORELLA, YANG SHENGTIAN, SIMMONS ROBERT L., CASAREZ ANTHONY, JONES ADRIANA K., LI CINDY, JANSEN JOHANNA M., MOSER HEINZ E.: "Size Matters and How You Measure It: A Gram-Negative Antibacterial Example Exceeding Typical Molecular Weight Limits", ACS INFECTIOUS DISEASES, AMERICAN CHEMICAL SOCIETY, US, vol. 5, no. 10, 11 October 2019 (2019-10-11), US , pages 1688 - 1692, XP055887741, ISSN: 2373-8227, DOI: 10.1021/acsinfecdis.9b00256

## Description

### FIELD OF THE INVENTION

This invention belongs to the medical field, and relates to novel monobactam compounds, processes for their preparation and said compounds for use as therapeutic agents. More particularly, the invention relates to amidine substituted monobactam compounds and said compounds for use as antibiotic agents for the treatment of bacterial infections.

### BACKGROUD OF THE INVENTION

The introduction of antibiotics for treatment of bacterial infections is one of the great medical achievements of 20^{th} century. Over the past few decades, however, bacterial resistance to multiple antibiotics has begun to emerge throughout the world, threatening the effectiveness of antibiotic therapy. The number of patients who are dying from untreatable nosocomial infections continues to grow. Therapeutic options are especially limited for infections due to multi-drug-resistant Gram-negative pathogens including Enterobacteriaceae and non-fermenters, a situation made worse by the fact that the pipelines of the pharmaceutical industry contain few compounds with promising resistance breaking profiles. New antibiotics are needed to combat the current and future threat of multidrug resistant bacteria.

*β*-lactams are the mostly widely used antibiotics for treatment of serious bacterial infections. These include cephalosporins, carbapenems, penicilins, and monobactams. As has been observed for other antibiotics classes, resistance to *β-*lactams has emerged. For most Gram-negative bacteria, this resistance is primarily driven by expression of *β*-lactamases, enzymes that hydrolyze *β*-lactam compounds. A vast array of more than 1000 *β*-lactamases and further resistance mechanisms severely endanger the mid-term usability of the current compounds in these subclasses. Especially extended-spectrum *β*-lactamases (ESBLs) and carbapenemases are important drivers of resistance. New *β*-lactams with resistance breaking properties are urgently needed to fill the gap.

Aztreonam as the single FDA approved monobactam is used worldwide for treatment of Gram-negative bacterial infections. However, aztreonam has poor activity against *Peseudomonas* and *Acinetobacter* strains. Because monobactams are inherently resistant to hydrolysis by metallo-lactamases, several companies have begun developing novel monobactam compounds for the treatment of infections caused by Gram-negative bacteria. Monobactam compounds comprising a siderophore moiety are disclosed by Basilea (WO 2007065288), Naeja Pharmaceuticals (WO 2002022613), Squibb & Sons (US 5290929, EP 531976, EP 484881). Pfizer re-investigated monocarbams, mono-cyclic *β*-lactams that carry a sulfonylaminocarbonyl activating group at the N1-position (WO 2010070523).

Recently, U.S Patent Application Publication No. US 2014/0275007 discloses oxamazin monobactam and their use as antibacterial agents. U.S Patent Application Publication No. US 2015/0266867 also discloses novel monobactam compounds for the use as antibacterial agents. International Patent Application Publication No. WO 2013/110643 discloses novel monobactam derivatives and their use as antibacterial agents. Additionally, International Patent Application Publication No. WO 2017/106064 discloses biaryl monobactam derivatives and their use as antibacterial agents, and International Patent Application Publication No. WO 2017/155765 discloses bicyclic aryl monobactam derivatives and their use as antibacterial agents.

The need for new antibiotics to overcome multidrug resistance continues. Compounds disclosed in this invention are designed to fill this medical need, through administration either on their own or in combination with a suitable *β-*lactamase inhibitor.

### DETAILED DESCRIPTION OF THE INVENTION

In one aspect, the present invention relates to novel amidine substituted monobactam compounds, which are useful in the treatment of bacterial infections in humans or animals either alone or in combination with *β*-lactamase inhibitors, such as clavulanic acid, tazobactam, sulbactam and other *β*-lactamase inhibitors belonging to the groups of lactam inhibitors, diazabicyclooctane inhibitors (avibactam and the like), transition state analog inhibitors and/or metallo-*β*-lactamase inhibitors.

In accordance with the present invention, there are provided (A) new compounds of general formula (I), (B) pharmaceutically acceptable salts of the compounds of formula (I), and (C) pharmaceutically acceptable solvates of the compounds of formula (I) and of their salts, and (D) deuterated compounds of compounds of (A), (B) and (C), (namely, (i) compounds of formula (I) modified in that they have been deuterated, (ii) pharmaceutically acceptable salts of the compounds of formula (I) modified in that they have been deuterated, (iii) pharmaceutically acceptable solvates of the compounds of formula (I) and of their salts modified in that they have been deuterated): 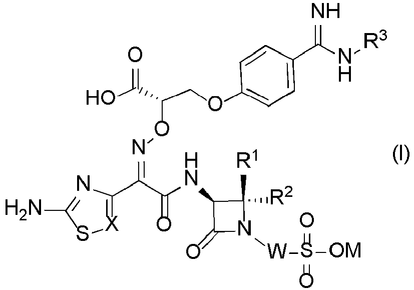 in which
- M: is hydrogen or a pharmaceutically acceptable salt forming cation,
- R¹ and R²: represent independently of one another hydrogen or methyl,
- W: represents a bond or O,
- X: represents CR⁴,
- R⁴: represents Cl,
- R³: represents hydrogen, amino, hydroxy, (C₁-C₆)-alkyl (preferably (C₁-C₄)-alkyl) or a 4-, 5- or 6-membered nitrogen-containing heterocycle, whereby alkyl may be substituted with a substituent selected from the group consisting of hydroxy, amino, carboxy, carbonyloxy, mono- or di-(C₁-C₆)-alkylamino (preferably di-(C₁-C₄)-alkylamino), -NH-CH(=NH),-NH-C(=NH)(NH₂), 5- or 6-membered nitrogen-containing heteroaryl and 5- or 6-membered nitrogen-containing heterocyclyl,
and the salts thereof, the solvates thereof and the solvates of the salts thereof.

Reference to specified compounds "modified in that they have been deuterated" refers to compounds prepared by modifying the specified compounds so that one or more hydrogen atoms in the compound have been replaced with or converted to deuterium.

Compounds of the invention are the compounds of formula (I) and the salts, solvates and solvates of the salts thereof, as well as the compounds which are encompassed by formula (I) and are mentioned hereinafter as exemplary embodiment(s), and the salts, solvates and solvates of the salts thereof, insofar as the compounds encompassed by formula (I) and mentioned hereinafter are not already salts, solvates and solvates of the salts

The compounds of the invention may, depending on their structure, exist in stereoisomeric forms (enantiomers, diastereomers). The invention therefore also encompasses the enantiomers or diastereomers and respective mixtures thereof. The stereoisomerically uniform constituents can be isolated in a known manner from such mixtures of enantiomers and/or diastereomers

If the compounds of the invention may occur in tautomeric forms, the present invention encompasses all tautomeric forms.

Salts preferred for the purposes of the present invention are physiologically acceptable salts of the compounds of the invention. Also encompassed however are salts which are themselves not suitable for pharmaceutical applications but can be used for example for the isolation or purification of the compounds of the invention.

Examples of pharmaceutically acceptable salts of the compounds of formula (I) include salts of inorganic bases like ammonium salts, alkali metal salts, in particular sodium or potassium salts, alkaline earth metal salts, in particular magnesium or calcium salts; salts of organic bases, in particular salts derived from benzylamine, octylamine, ethanolamine, diethanolamine, cyclohexylamine, diethylamine, triethylamine, ethylenediamine, procaine, morpholine, pyrroline, piperidine, N-ethylpiperidine, N-methylmorpholine, piperazine as the organic base; or salts with basic amino acids, in particular lysine, arginine, histidine and ornithine.

Examples of pharmaceutically acceptable salts of the compounds of formula (I) also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, benzenesulfonates or toluenesulfonates; or salts with acidic amino acids, in particular aspartate or glutamate.

Solvates for the purposes of the invention refer to those forms of the compounds of the invention which in the solid or liquid state form a complex by coordination with solvent molecules. Hydrates are a specific form of solvates in which the coordination takes place with water.

In the context of this invention the substituents have the following definitions unless specified otherwise.

The term alkyl refers to straight-chain or branched C₁-C₆ alkyl, preferably C₁-C₄ alkyl, such as in particular methyl, ethyl, propyl, butyl, isopropyl, isobutyl and tert-butyl.

The term heteroaryl refers to cyclic heteroaromatic groups with 5-8 ring atoms, preferably with 5-6 ring atoms, and with up to 4, preferably with up to 2, heteroatoms selected from the group consisting of N, O, S, in which N can also form an N-oxide. Preferred are monocyclic heteroaryl groups with 5-6 ring atoms including up to 2 hetero atoms selected from the group consisting of N, O and S, such as in particular benzothiophene, furane, benzofurane, pyrrole, thiophene, pyridine, pyrazine, pyrimidine, pyridazine, indole, isoindole, purine, quinoline pyrazole, imidazole, thiazole, thiadiazole, oxazole, isoxazole, or isoquinoline. Many other suitable heteroaryl groups for the purpose of the invention are known to the person skilled in the art or can be readily found in the literature.

The term heterocyclyl refers to saturated or partially unsaturated heterocyclic groups with 4-10 ring atoms, preferably with 5-6 ring atoms, and with up to 3, preferably with up to 2, heteroatoms selected from the group consisting of N, O, S, SO and SO₂, in which N can also form an N-oxide. Preferred are saturated monocyclic heterocyclyl groups with 5-6 ring atoms including up to 2 hetero atoms selected from the group consisting of N, O and S, such as in particular pyrroline, pyrrolidine, imidazolidine, thiazolidine, imidazoline, tetrahydrofuran, tetrahydrothiophene, piperidine, pyran, tetrahydropyran, thiopyran, tetrahydrothiopyran, morpholine, thiomorpholine, piperazine, piperidazine. Many other suitable heterocyclyl groups for the purpose of the invention are known to the person skilled in the art or can be readily found in the literature.

The term halogen refers to fluorine, chlorine, bromine or iodine; preferably fluorine or chlorine.

The term carboxy refers to a carboxylic acid group, i.e. a-COOH group.

The term carbonyloxy refers to a carbonyl group linked via an oxygen.

The present invention relates to compounds of formula (I) in which
- M: is hydrogen or a pharmaceutically acceptable salt forming cation,
- R¹ and R²: represent methyl,
- W: represents O,
- X: represents CR⁴,
- R⁴: represents Cl,
- R³: represents aminoethyl, azetidine, pyrrolidine or piperidine, and the salts thereof, the solvates thereof and the solvates of the salts thereof.

Also disclosed but not claimed are compounds of formula (I) in which
- M: is hydrogen or a pharmaceutically acceptable salt forming cation,
- R¹ and R²: represent methyl,
- W: represents O,
- X: represents N
- R³: represents aminoethyl, azetidine, pyrrolidine or piperidine, and the salts thereof, the solvates thereof and the solvates of the salts thereof.

Also disclosed are compounds of formula (I) having the following structure: Only the 5-chlorothiazoles thereof form part of the invention. 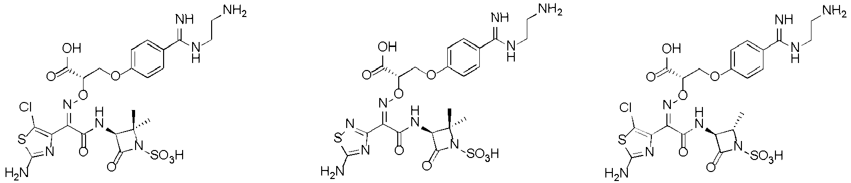 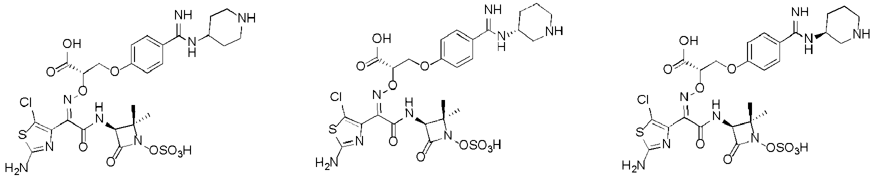 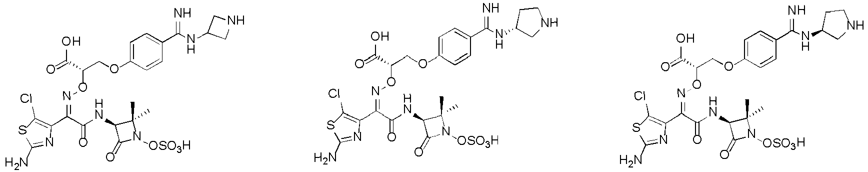 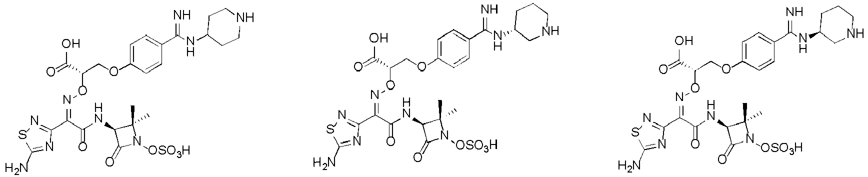 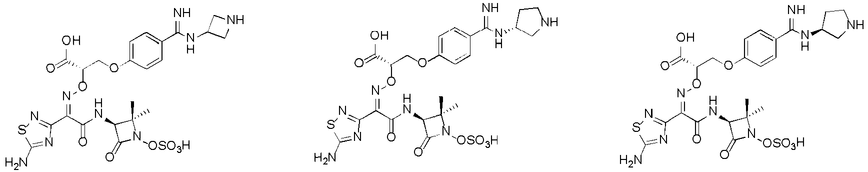 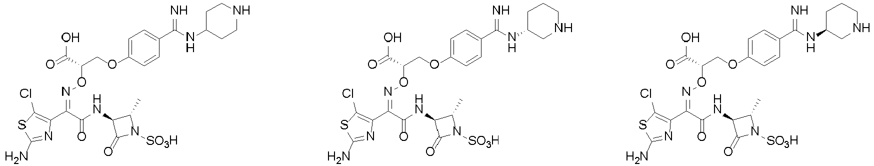 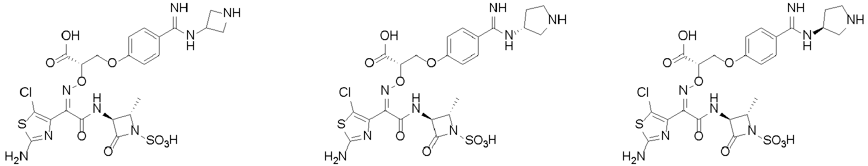 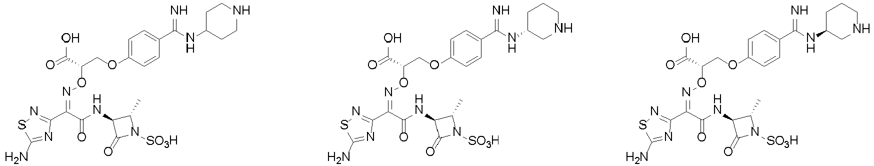 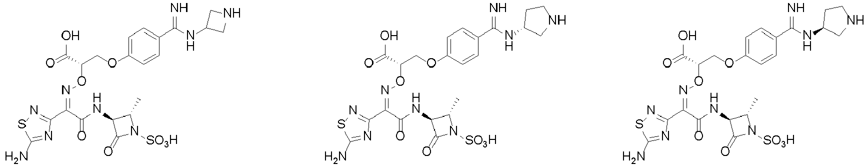 and the salts thereof, the solvates thereof and the solvates of the salts thereof.

The present invention also relates to methods for the preparation of compounds of formula (I). The compounds of the present invention may be prepared by removing the protecting group from compounds of formula (II) 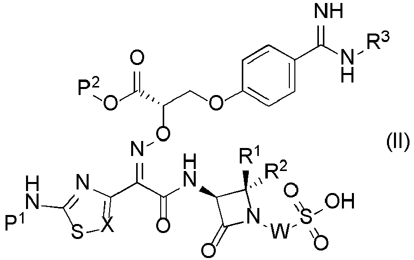 in which P¹ and P² represent independently a protecting group and R¹, R², R³, W and X are defined as above, under acidic conditions.

Acidic conditions may involve treating the compounds of formula (II) with trifluoroacetic acid, formic acid, acetic acid, or hydrochloric acid at temperatures ranging from 0°C to room temperature for a time ranging from several minutes to hours, preferably with 90% formic acid or trifluoroacetic acid at a temperature of 30-60 °C for 30-60 min.

Compounds of formula (II) can be synthesized by reacting compounds of formula (III) 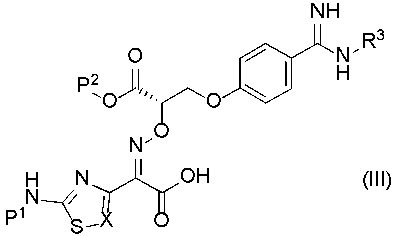
in which P¹ and P² represent independently a protecting group and R³ and X are defined as above,
with compounds of formula (IV) 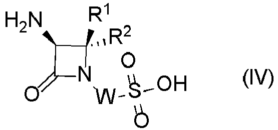
in which R¹, R² and W are as defined above.

The reaction generally takes place in inert solvents in the presence of a coupling reagent and where applicable with addition of a base at a temperature ranging from -20°C to 80°C for 1-24 hours, preferably at a temperature of 20-30°C overnight. Inert solvents are for example *N*,*N-*dimethylformamide (DMF), *N*,*N*-dimethylacetamide (DMA), *N*-methylpyrrolidin-2-one (NMP), dichloromethane (DCM), tetrahydrofuran (THF), 1,4-dioxane, acetonitrile as well as mixtures of the aforementioned solvents. A preferred solvent is N,N-dimethylformamide.

Suitable coupling reagents are for example 1,2-oxazolium compounds such as 2-ethyl-5-phenyl-1,2-oxazolium-3-sulfate or 2-tert-butyl-5-methyl-isoxazo-lium-perchlorate, or carbonyl compounds such as carbonyldiimidazole (CDI), or acylamino compounds such as 2-ethoxy-1-ethoxycarbonyl-1,2-dihydrochinoline, or propanphosphonic acid anhydride, or isobutylchloroformate, or bis-(2-oxo-3-oxazolidinyl)-phosphorylchloride, or O-(benzotriazol-1-yl)-*N*,*N*,*N'*,*N'*-tetramethyluro-niumhexafluorophosphate (HBTU), 2-(2-oxo-1-(2H)-pyridyl)-1,1,3,3-tetramethyluroni-umtetrafluoroborate (TPTU) or O-(7-azabenzotriazol-1-yl)- *N*,*N*,*N'*,*N'*-tetramethyl-uroniumhexafluorophosphate (HATU), or 1-hydroxybenzotriazole (HOBt), or benzo-triazol-1-yloxytris(dimethylamino)-phosphoniumhexafluorophosphate (BOP), or benzotriazol-1-yloxytris(pyrrolidino)-phosphoniumhexafluorophosphate (PyBOP), or carbodiimides such as *N*,*N'*-diethyl-, *N*,*N'*-dipropyl-, *N*,*N'*-diisopropyl-, *N*,*N'*-dicyclohexylcarbodiimide (DCC), *N*-(3-dimethylaminoisopropyl)-*N*'-ethylcarbodiimid-hydrochloride (EDC), *N*-cyclohexylcarbodiimid-*N'*-propyloxymethyl-polystyrole (PS-Carbodiimide) or *N*-hydroxysuccinimide as well as mixtures of the above mentioned coupling reagents with or without the addition of base. In the certain case both inorganic and organic bases may be used. Suitable bases are for example carbonates and bicarbonates, triethylamine, diisopropylethylamine, *N*-methylmorpholine, *N-*methylpiperidine or 4-dimethylaminopyridine. Preferably, reactions are carried out with a mixture of a carbodiimide and 1-hydroxybenzotriazole with or without the addition of sodium bicarbonate as base.

Compounds of formula (III) can be prepared by reacting compounds of formula (V) 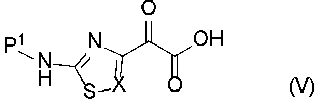
in which P¹ represents a protecting group, and X is as defined above,
with compounds of formula (VI) 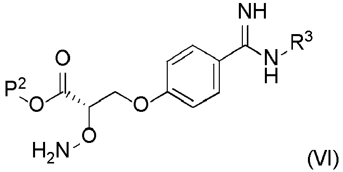
in which P² represents a protecting group, and R³ is defined as above.

The reaction generally takes place in protic solvents or in solvent mixtures containing at least one protic solvent at a temperature ranging from 0°C to 100°C for 1-24 hours. Suitable protic solvents are for example methanol, ethanol, isopropanol. Solvents suitable to form mixtures are for example dichloromethane, trichloromethane, tetrahydrofuran, 1,4-dioxane, acetonitrile and *N,N-*dimethylformamide. Preferably, the reaction is carried out in a mixture of anhydrous ethanol and chloroform at 20-30°C overnight.

Compounds of formula (IV) can be synthesized according to WO2013110643A1. 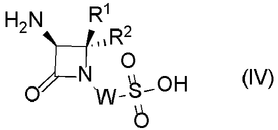 in which R¹, R² and W are as defined above.

Compounds of formula (V) can be synthesized according to the following literature references: Bioorg. Med. Chem., 2007, 38 (21), 6716-6732; US20120264727A1 or by adapting the referenced procedures in a way known to a person skilled in art. 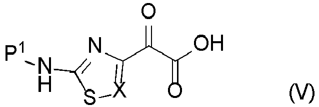 in which P¹ represents a protecting group, and X is defined as above.

The compounds of the invention show a valuable range of pharmacological effects which could not have been predicted.

They are therefore suitable for use as medicaments for the treatment and/or prophylaxis of diseases in humans and animals.

The compounds of the present invention are distinguished in particular by an advantageous range of antibacterial effects.

The present invention further relates to the compounds of the invention for use in the treatment and/or prophylaxis of diseases caused by bacteria, especially Gram-negative bacteria.

The present invention further relates to the compounds of the invention for use in the treatment and/or prophylaxis of diseases, especially of diseases mentioned below.

The present invention further relates to the use of the compounds of the invention for the manufacture of a medicament for the treatment and/or prophylaxis of diseases, especially of bacterial infections and in particular diseases mentioned below.

The present invention further relates to the compounds of the invention for use in a method for the treatment and/or prophylaxis of diseases, especially of bacterial infections and in particular diseases mentioned below, using a therapeutically effective amount of the compounds of the invention.

The compounds of the invention exhibit an antibacterial spectrum against Gram-negative bacteria and selected Gram-positive bacteria combined with low toxicity. Compounds of this invention are particularly useful in human and veterinary medicine for the prophylaxis and treatment of local and systemic infections which are caused for example by the following pathogens or by mixtures of the following pathogens:

Aerobic Gram-positive bacteria: including but not limited to *Streptococcus spp. (S. pneumoniae, S. pyogenes, S. agalactiae, Streptococcus group C and G)* , *Staphylococcus spp. (S. aureus) as well as Bacillus spp.* and *Listeria monocytogenes.*

Aerobic Gram-negative bacteria: *Enterobacteriaceae,* including but not limited to *Escherichia* spp. *(E. coli), Klebsiella* spp. (*K. pneumoniae, K. oxytoca*), *Citrobacter* spp. (*C*. *freundii, C. diversus*), *Morganella morgannii, Hafnia alvei, Serratia* spp. (*S. marcescens*)*, Enterobacter* spp. (*E. cloacae, E. aerogenes*), *Proteus* spp. (*P. mirabilis, P. vulgaris, P. penneri*), *Providencia* spp. (*P. stuartii, P. rettgeri*), *Yersinia* spp. (*Y. enterocolitica, Y. pseudotuberculosis*) *Salmonella* spp., *Shigella* spp. and also non-fermenters including but not limited to *Pseudomonas* spp. (*P. aeruginosa*), *Burkholderia* spp. (*P. cepacia*), *Stenotrophomonas maltophilia,* and *Acinetobacter* spp. (*A. baumannii, Acinetobacter* gen. sp. 13TU, *Acinetobacter* gen. sp. 3) as well as *Bordetella spp.* (*B. bronchiseptica*), *Moraxella catarrhalis* and *Legionella pneumophila*; furthermore, *Aeromonas* spp., *Haemophilus* spp. (*H. influenzae*)*, Neisseria spp.* (*N. gonorrhoeae, N. meningitidis*) as well as *Alcaligenes* spp. (including *A. xylosoxidans*)*, Helicobacter pylori, Vibro* spp. (*V*. *cholerae), Campylobacter jejuni* and. *Pasteurella* spp. (*P. multocida*).

Moreover, the antibacterial spectrum comprises also strictly anaerobic bacteria including but not limited to *Peptostreptococcus spp.*(*P. anaerobius*)*, Bacteroides spp.* (*B. fragilis*), *Prevotella* spp., *Brucella* spp. (*B. abortus*), *Clostridium* spp. (*Clostridium perfringens*) and *Porphyromonas* spp.,

The above listing of pathogens is merely exemplary and in no way to be regarded as limiting. Examples of diseases which may be caused by the said pathogens and which may be prevented, improved or cured by the compounds according to the invention are, for example:

Respiratory tract infections such as lower respiratory tract infections, lung infection in cystic fibrosis patients, acute exacerbation of chronic bronchitis, community acquired pneumoniae (CAP), nosocomial pneumonia (including ventilatorassociated pneumonia (VAP)), diseases of the upper airways, diffuse panbronchiolitis, tonsillitis, pharyngitis, acute sinusitis and otitis including mastoiditis; urinary tract and genital infections for example cystitis, uretritis, pyolonephritis, endometritis, prostatitis, salpingitis and epididymitis; ocular infections such as conjunctivitis, corneal ulcer, iridocyclitis and post-operative infection in radial keratotomy surgery patients; blood infections, for example septicaemia; infections of the skin and soft tissues, for example infective dermatitis, infected wounds, infected burns, phlegmon, folliculitis and impetigo; bone and joint infections such as osteomyelitis and septic arthritis; gastrointestinal infections, for example dysentery, enteritis, colitis, necrotising enterocolitis and anorectal infections; intraabdominal infections such as typhoid fever, infectious diarrhea, peritonitis with appendicitis, pelviperitonitis, and intra-abdominal abscesses; infections in the oral region for example infections after dental operations; other infections for example, meliodosis, infectious endocarditis, hepatic abscesses, cholecystitis, cholangitis, mastitis as well as meningitis and infections of the nervous systems.

In addition to humans, bacterial infections can also be treated in animals, such as horse, primates, pigs, ruminants (sheep, cow, goat), dog, cat, poultry (such as hen, turkey, quail, pigeon, ornamental birds) as well as productive and ornamental fish, amphibians and reptiles.

The compounds of the invention may act systemically and/or locally. For this purpose, they can be administered in a suitable way, such as, for example, parenterally, pulmonarily, nasally, sublingually, lingually, buccally, rectally, dermally, transdermally, conjunctivally, or as an implant or stent.

For these administration routes the compounds of the invention can be administered in suitable administration forms.

Parenteral administration can take place with an absorption step (e.g. intramuscular, subcutaneous, intracutaneous, percutaneous, or intraperitoneal) or avoidance of an absorption step (e.g. intravenous, intraarterial, intracardiac, intraspinal or intralumbar). Administration forms suitable for parenteral administration are, inter alia, preparations for injection and infusion in the form of solutions, suspensions, emulsions, sterile powders or lyophilizates.

Suitable for the other administration routes are, for example, pharmaceutical forms for inhalation (inter alia powder inhalers, nebulizers), solutions, nasal drops, sprays; tablets, films/wafers or capsules, for lingual, sublingual or buccal administration, suppositories, preparations for ears or eyes, vaginal capsules, aqueous suspensions (lotions, shaking mixtures), ointments, creams, lipophilic suspensions, transdermal therapeutic systems (such as for example patches), pastes, milk, foams, dusting powders, stents or implants.

The compounds of the invention can be converted into the stated administration forms. This can take place in a manner known per se by mixing with inert, non-toxic, pharmaceutically acceptable excipients. These excipients include inter alia carriers (for example microcrystalline cellulose, lactose, mannitol), emulsifiers and dispersants or wetting agents (for example sodium dodecyl sulfate, polyoxysorbitan oleate), solvents (e.g. liquid polyethylene glycols), binders (for example polyvinylpyrrolidone), synthetic and natural polymers (for example albumin), colors (e.g. inorganic pigments such as, for example, iron oxides), stabilizers (e.g. antioxidants such as, for example, ascorbic acid), and odor corrigents and/or taste.

The present invention further relates to medicaments which comprise at least one compound of the invention, usually together with one or more inert, non-toxic, pharmaceutically acceptable excipients, as well as to their use for the aforementioned purposes.

The present invention further relates to medicaments which comprise at least one compound of the invention in combination with at least one *β*-lactamase inhibitor, as well as to their use for the aforementioned purposes.

Examples for the further active compound include *β*-lactamase inhibitors.

Examples for suitable *β*-lactamase inhibitors to be used in combination with the compounds of the invention include clavulanic acid, tazobactam, sulbactam, avibactam, relebactam, vaborbactam.

The minimum amount of the compounds of the invention to be administered is a therapeutically effective amount. The term "therapeutically effective amount" means the amount of compound which prevents the onset of, alleviates the symptoms of, stops the progression of, and/or eliminates a bacterial infection in humans or animals.

Typically, an effective dosing schedule of the compounds of the invention for adults is about 50 mg to about 3000 mg of a compound of formula (I) in a single dose; in another embodiment, an effective single dose is about 100 mg to about 2000 mg. In another embodiment, an effective single dose is about 500 mg to about 1200 mg. Typically the dosages are given 1 to 4 times per day. In one embodiment, the dosages are given 3 times per day. In some cases, it may be necessary to use dosages outside these limits.

It may nevertheless be necessary where appropriate to deviate from the stated amounts, in particular as a function of body weight, administration route, individual response to the active ingredient, type of preparation and time or interval over which administration takes place. Thus, in some cases it may be sufficient to make do with less than the aforementioned minimum amount, whereas in other cases the upper limit mentioned must be exceeded. In the case of an administration of larger amounts, it may be advisable to distribute these in a plurality of single doses over the day.

The percentage data in the following tests and examples are, unless indicated otherwise, percentages by weight; parts are parts by weight. Solvent ratios, dilution ratios and concentration data of liquid/liquid solutions are based in each case on volume. The statement "w/v" means "weight/volume". Thus, for example, "10% w/v" means: 100 ml of solution or suspension contain 10 g of substance.

### Examples

### Abbreviations

- NMR:: nuclear magnetic resonance
- δ:: chemical shift in ppm
- br s:: broard singlet in NMR
- CDCl₃:: deuterated chloroform
- Hz:: hertz
- MHz:: megahertz
- J:: coupling constant in NMR
- d:: doublet in NMR
- dd:: doublet of doublet in NMR
- q:: quartet in NMR
- s:: singlet in NMR
- t:: triplet in NMR
- m:: multiplet in NMR
- TMS:: tetramethylsilane
- DCM:: dichloromethane
- DMF:: *N*,*N*-dimethylformamide
- DMSO:: dimethyl sulfoxide
- EtOAc:: ethyl acetate
- t-BuOH:: tert-butyl alcohol
- TFA:: trifluoroacetic acid
- THF:: tetrahydrofuran
- PE:: petroleum ether
- DCC:: *N*,*N'*-dicyclohexylcarbodiimide
- HOBt:: 1-hydroxybenzotriazole
- TMEAD:: tetramethylethylenediamine
- TLC:: thin layer chromatography
- MS:: mass spectrometry
- ES⁻:: negative ion mode in electrospray ionization mass spectrometry
- ES⁺:: positive ion mode in electrospray ionization mass spectrometry
- HPLC:: high performance liquid chromatography
- L:: liter(s)
- M:: molarity
- mg:: milligram(s)
- min:: minute(s)
- mL:: milliliter(s)
- mmol:: millimole(s)
- mol:: mole(s)
- g:: gram(s)
- h:: hour(s)
- N:: normality

### Analytical Methods

All ¹H and ¹⁹F NMR spectra were recorded on a Bruker AVANCE NEO 400 NMR operating at 400 MHz for ¹H, and 376 MHz for ¹⁹F respectively. NMR data is recorded in chemical shifts relative to tetramethylsilane (TMS) as internal standard. NMR spectra were run either in CDCl₃ containing 0.05 % TMS, CD₃OD containing 0.05 % TMS, D₂O or DMSO-*d*₆ containing 0.03 % TMS.

Preparative HPLC was performed on a Agilent 1260 Infinity II System on Agilent 10 prep-C18 250x21.2 mm column, using an acetonitrile/aqueous 0.1 % trifluoroacetic acid gradient, or an acetonitrile/aqueous 0.1 % formic acid gradient at 22°C.

Mass spectra were recorded on a Agilent 1260 Infinity II System using either ES⁻ or ES⁺ ionization modes.

Column chromatography was performed using Qingdao Inc. Silica Gel: CC Grade (230 - 400 Mesh).

Commercial solvents and reagents were generally used without further purification. All products were dried before characterization and use in subsequent synthetic steps.

### General Synthetic Methods

### 1. Synthesis of the β-lactam building blocks

### 1.1 (3S)-3-Amino-4,4-dimethyl-1-(sulfooxy)azetidin-2-one (1_1)

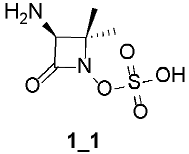

Compound 1_1 was synthesized according to WO2013110643A1.

H¹ NMR (400 MHz, DMSO- *d*₆): δ 1.42 (s, 3 H), 1.43 (s, 3 H), 4.15 (s, 1 H), 8.80 (br. s, 2 H).

### 1.2 (3S,4S)-3-Amino-4-methyl-2-oxoazetidine-1-sulfonic acid (1_2)

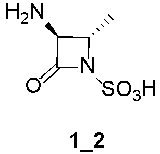

Compound **1_2** was synthesized according to David M. Floyd, Alan W. Fritz, Josip Pluscec, Eugene R. Weaver, Christopher M. Cimarusti J. Org. Chem., 1982, 47 (26), 5 160-5 167.

H¹ NMR (400 MHz, DMSO- *d*₆): δ 1.39 (d, *J* = 6.2 Hz, 3 H), 3.75-3.81 (m, 1 H), 4.00 (d, *J* = 4.0 Hz, 1 H), 8.66 (br. s, 3 H).

### 2. Synthesis of amino-thiazole and amino-thiadiazole building blocks

### 2.1 2-(2-((tert-Butoxycarbonyl)amino)-5-chlorothiazol-4-yl)-2-oxoacetic acid (2_1)

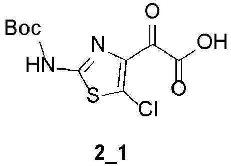

Compound **2_1** was synthesized according to Bioorg. Med. Chem., 2007, 38 (21), 6716-6732.

¹H NMR (400 MHz, DMSO-*d*₆): *δ* 1.47 (s, 9 H), 12.1 (s, 1 H), (COOH not shown).

### 2.2 {5-[(tert-Butoxycarbonyl)amino]-1,2,4-thiadiazol-3-yl}(oxo)acetic acid (2_2)

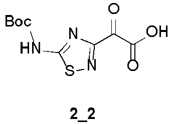

Compound **2_2** was synthesized according to US20120264727A1.

¹H NMR (400 MHz, DMSO-*d*₆) *δ* 1.51 (s, 9 H), 12.81 (s, 1 H), (COOH not shown).

### 3. Synthesis of amidine side chains

### 3.1 tert-Butyl (S)-4-(4-(2-(aminooxy)-3-(benzhydryloxy)-3-oxopropoxy)benzimidamido)piperidine-1-carboxylate (3_1_8)

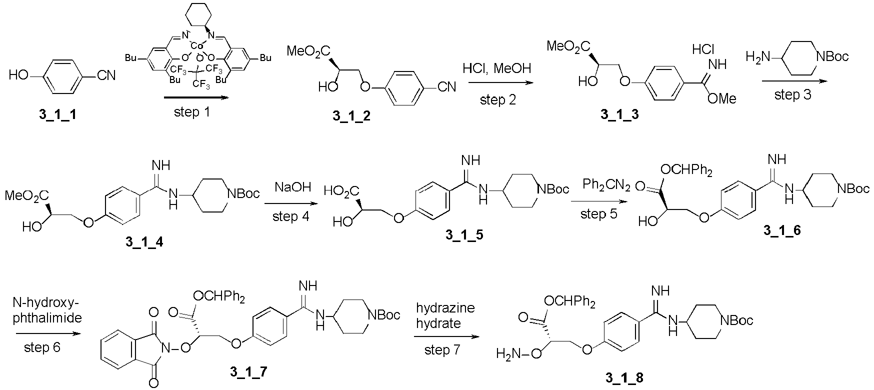

### Step 1: Methyl (R)-3-(4-cyanophenoxy)-2-hydroxypropanoate (3_1_2)

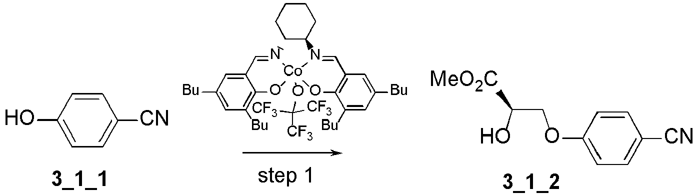

A mixture of a Co(III)-catalyst (7.0 g, 8.4 mmol, ref: J. Am. Chem. Soc. 1999, 121, 6086-6087), and 4Å molecular sieves (10 g) in tert-butyl methyl ether (50 mL) was treated with methyl (R)-oxirane-2-carboxylate (45.0 g, 441 mmol) and 4-hydroxybenzonitrile **3_1_1** (26.3 g, 220 mmol). The reaction mixture was stirred at room temperature for two days and filtered through a pad of Celite. The filtrate was concentrated to give a dark brown residue. The residue was purified by column chromatography to afford compound **3_1_2** (79.0 g, 357 mmol, 81.1 % yield) as a brown oil.

¹H NMR (400 MHz, DMSO-*d*₆): *δ* 3.68 (s, 3 H), 4.26 (m, 2 H), 4.49 (m, 1 H), 5.96 (s, 1 H), 7.11 (d, *J* = 8.96 Hz, 2 H), 7.77 (d , *J* = 9.04 Hz, 2 H).

### Step 2: (R)-2-Hydroxy-3-(4-methoxycarbonimidoyl-phenoxy)-propionic acid methyl ester hydrochloric acid salt (3_1_3)

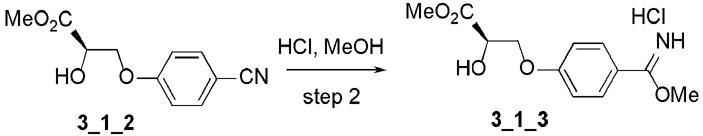

A mixture of compound **3_1_2** (36.6 g, 165 mmol) in anhydrous methanol (160 mL) was cooled to 0°C in a sealed vessel. Anhydrous hydrogen chloride gas was bubbled through the solution until the mixture was saturated. The flask was sealed and stirred at 0°C to room temperature for 18 hours to give a suspension. The solid was collected to give desired compound **3_1_3** (29 g) after diethyl ether wash. The mother liq. was concentrated to give a suspension again, the solid was collected to give additional desired compound (9.89 g) after diethyl ether wash (totally 38.9 g of desired compound obtained, 81.5 % yield) as light green solid.

1HNMR (400 MHz, DMSO- *d*₆): *δ* 3.66 (s, 3 H), 4.24 (s, 3 H), 4.27-4.31 (m, 2 H), 4.48 (t, *J* = 4.4 Hz, 1 H), 7.16 (d, *J* = 9.1 Hz, 2 H), 8.09 (d, *J* = 9.1 Hz, 2 H).

### Step 3: tert-Butyl (R)-4-(4-(2-hydroxy-3-methoxy-3-oxopropoxy)benzimidamido)piperidine-1-carboxylate (3_1_4)

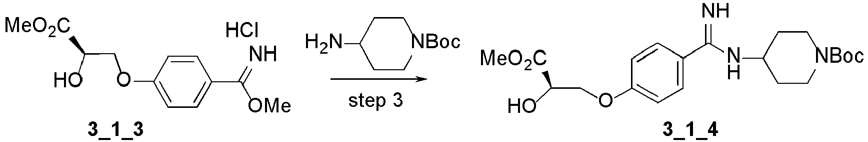

To a mixture of compound **3_1_3** (1.0 g, 3.45 mmol) in anhydrous methanol (3 mL) were added triethylamine (0.38 g, 3.8 mmol) and *tert*-butyl 4-aminopiperidine-1-carboxylate (0.69 g, 3.45 mmol) at 0 °C and the resulting mixture was stirred at room temperature overnight. The reaction mixture was concentrated to dryness to afford a residue. The residue was purified by column chromatography to afford compound **3_1_4** (1.0 g, 69% yield) as a white solid.

¹HNMR (400 MHz, DMSO-*d*₆): *δ* 1.38 (s, 9 H), 1.54-1.43 (m, 2 H), 1.82-1.93 (m, 2 H), 2.60-2.90 (m, 2 H), 3.64 (s, 3H), 3.90-4.00 (m, 3 H), 4.12-4.28 (m, 2 H), 4.44-4.50 (m, 1 H), 5.98 (s, 1 H), 7.22 (d, *J* = 8.8 Hz, 2 H), 7.69 (d, *J* = 8.8 Hz, 2 H).

### Step 4: (R)-3-(4-(N-(1-(tert-Butoxycarbonyl)piperidin-4-yl)carbamimidoyl)phenoxy)-2-hydroxypropanoic acid (3_1_5)

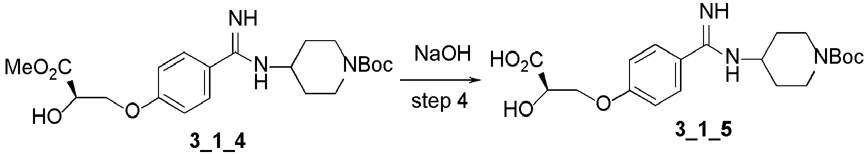

To the solution of compound **3_1_4** (1.0 g, 2.37 mmol) in tetrahydrofuran (2 mL) was added a solution of sodium hydroxide (0.2 g, 4.74 mmol) in water (1 mL) at 0 °C, and then stirred at room temperature for 5 hours. Tetrahydrofuran was removed, the reaction mixture was neutralized to pH 5 using 1 N HCl. The mixture was lyophilized to afford crude compound **3_1_5** (0.97 g, containing NaCl salt) as a white solid, which was directly used for next step without further purification.

¹H NMR (400 MHz, DMSO-*d*₆): *δ* 1.41 (s, 9 H), 1.49 (br s, 2 H), 1.92 (br s, 2 H), 2.84 (br s, 2 H), 3.86 (br s, 1 H), 4.00 (br s, 4 H), 4.26 (d, *J* = 9.8 Hz, 1 H), 7.12 (s, 2 H), 7.74 (s, 2 H).

### Step 5: tert-Butyl (R)-4-(4-(3-(benzhydryloxy)-2-hydroxy-3-oxopropoxy)benzimidamido)piperidine-1-carboxylate (3_1_6)

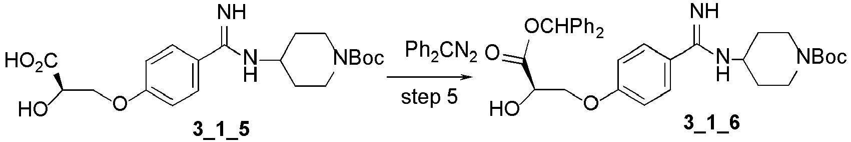

To a solution of compound **3_1_5** (0.97 g, 2.37 mmol) in MeOH (2 mL) was slowly added a solution of diazo(diphenyl)methane (Ph₂CN₂, 0.42 g, 3.56 mmol) in DCM (1.5 mL). The resulting mixture was stirred at room temperature overnight and concentrated to dryness. The residue was purified by column chromatography to give product **3_1_6** (1.1 g, 80% yield in two steps) as a white solid.

¹H NMR (400 MHz, DMSO-*d*₆): *δ* 1.39 (s, 9 H), 1.42-1.52 (m, 2 H), 1.87-1.92 (m, 2 H), 2.82 (br s, 2 H), 3.90-4.00 (m, 3 H), 4.30-4.42 (m, , 2 H), 4.62-4.65 (m, 1 H), 6.48 (d, *J* = 7.6 Hz ,1 H), 6.84 (s, 1 H), 7.09 (d, *J* = 8.8 Hz, 2 H), 7.22-7.41 (m, 10 H), 7.69 (d, *J* = 8.8 Hz, 2 H), 9.10 (br s, 1 H), 9.38 (br s, 1 H). LC-MS analysis: [M+H]⁺ = 574.1.

### Step 6: tert-Butyl (S)-4-(4-(3-(benzhydryloxy)-2-((1,3-dioxoisoindolin-2-yl)oxy)-3-oxopropoxy)benzimidamido)piperidine-1-carboxylate (3_1_7)

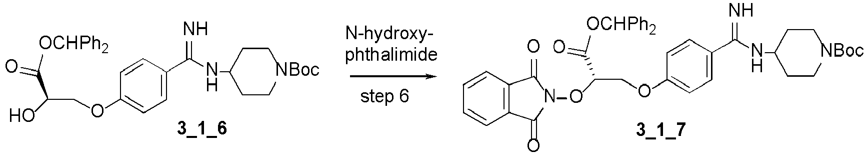

To a solution of compound **3_1_6** (1.2 g, 2.1 mmol), N-hydroxyphthalimide (1.03 g, 6.3mmol mmol) and triphenylphosphine (1.7 g, 6.3 mmol) in anhydrous tetrahydrofuran (5 mL) was added a solution of diethyl azodicarboxylate (1.09 g, 6.3 mmol) dropwise at 0 °C. The resulting mixture was stirred at room temperature overnight. After evaporation of the tetrahydrofuran, the crude product was purified by column chromatography to give compound **3_1_7** (1.0 g, 66.7% yield) as a white solid.

¹H NMR (400 MHz, DMSO-*d*₆): *δ* 1.42 (s, 9 H), 1.44-1.53 (m, 2 H), 1.92-1.96 (m, 2 H), 2.82 (br s, 2 H), 3.92-4.16 (m, 3 H), 4.68 (br s, 2 H), 5.48 (br s, 1 H), 6.98 (s, 1 H), 7.11 (d, *J* = 8.6 Hz, 2 H), 7.26-7.48 (m, 10 H), 7.75 (d, *J* = 8.6 Hz, 2 H), 7.86 (s, 4 H), 9.24 (br s, 1 H), 9.40 (b. s, 1 H). LC-MS analysis: [M+H]⁺ = 719.1.

### Step 7: tert-Butyl (S)-4-(4-(2-(aminooxy)-3-(benzhydryloxy)-3-oxopropoxy)benzimidamido)piperidine-1-carboxylate (3_1_8)

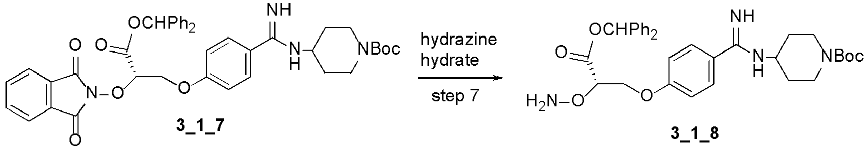

To the solution of compound **3_1_7** (0.33 g, 0.45 mmol) in anhydrous ethanol (5 mL) was added hydrazine monohydrate (23 µL, 0.52 mmol) at 0 °C. The resulting mixture was stirred at room temperature for 3.5 hours, filtered off and washed with ethanol (2x5 mL). The filtrate was concentrated to dryness and the residue was suspended with CH₂Cl₂ (10 mL), filtered off and rinsed with CH₂Cl₂ (2x3 mL). The filtrate was concentrated to give compound **3_1_8** (0.26 g, quant. yield) as a pale yellow foam, which was used for next step without further purification.

¹H NMR (400 MHz, DMSO-*d*₆): *δ* 1.42 (s, 9 H), 1.42-1.54 (m, 2 H), 1.87-1.97 (m, 2 H), 2.72-2.94 (m, 2 H), 3.82-4.06 (m, 3 H), 4.30-4.49 (m, 2 H), 4.60-4.68 (m,1 H), 6.91 (s, 1 H), 7.10 (d, *J* = 8.8 Hz, 2 H), 7.25-7.47 (m, 10 H), 7.71 (d, *J* = 8.8 Hz, 2 H), 9.15 (brs, 4 H). LC-MS analysis: [M+H]⁺ = 561.2.

Following the procedure detailed above under 3.1 but using the diffrent amines shown in the scheme below instead of tert-butyl 4-aminopiperidine-1-carboxylate in step 3 of the synthesis, compounds **3_2_8** to **3_6_8** were prepared.

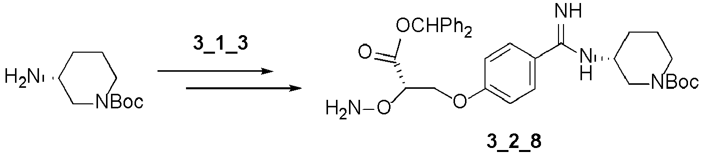

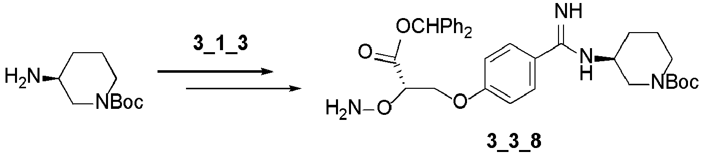

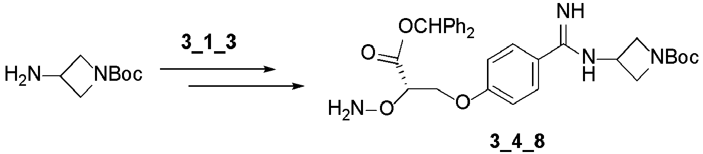

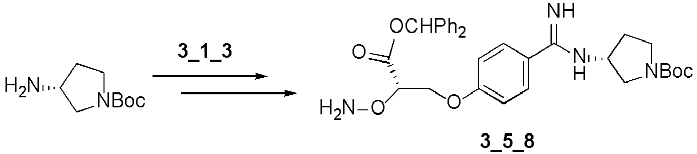

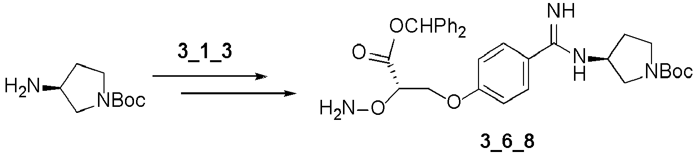

### 3.2 tert-Butyl (R)-3-(4-((S)-2-(aminooxy)-3-(benzhydryloxy)-3-oxopropoxy)benzimidamido)piperidine-1-carboxylate (3_2_8)

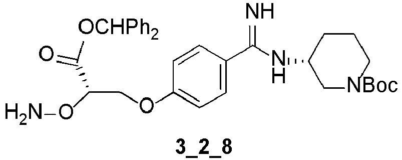

¹H NMR (400 MHz, DMSO-*d*₆): *δ*1.38 (s, 9 H), 1.43-1.49 (m, 1 H), 1.52-1.60 (m, 1 H), 1.72-1.82 (m, 1 H), 1.96-2.03 (m, 1 H), 2.91-3.04 (m, 1 H), 3.33-3.39 (m, 3 H), 3.75-3.82 (m, 1 H), 4.36-4.47 (m, 2 H), 4.62-4.65 (m, 1 H), 6.45 (s, 2 H), 6.90 (s, 1 H), 7.07 (d, *J* =8.9 Hz, 2 H), 7.23-7.32 (m, 4 H), 7.33-7.40 (m, 4 H), 7.42-7.45 (m, 2 H), 7.69 (d, *J* =8.9 Hz, 2 H). LC-MS analysis:[M+H]⁺ = 589.3.

### 3.3 tert-Butyl (S)-3-(4-((S)-2-(aminooxy)-3-(benzhydryloxy)-3-oxopropoxy)benzimidamido)piperidine-1-carboxylate (3_3_8)

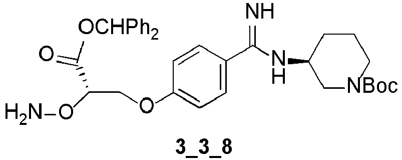

¹H NMR (400 MHz, DMSO-*d*₆): *δ* 1.39 (s, 9 H), 1.43-1.49 (m, 1 H), 1.51-1.61 (m, 1 H), 1.74-1.82 (m, 1 H), 1.96-2.04 (m, 1 H), 2.92-3.04 (m, 1 H), 3.40-3.47 (m, 3 H), 3.74-3.83 (m, 1 H), 4.37-4.44 (m, 2 H), 4.64 (t, *J* = 3.9 Hz, 1 H), 6.46 (s, 2 H), 6.90 (s, 1 H), 7.09 (d, *J* = 8.7 Hz, 2 H), 7.22-7.32 (m, 4 H), 7.33-7.40 (m, 4 H), 7.41-7.47 (m, 2 H), 7.69 (d, *J* = 8.7 Hz, 2 H). LC-MS analysis:[M+H]⁺ = 589.3.

### 3.4 tert-Butyl (S)-3-(4-(2-(aminooxy)-3-(benzhydryloxy)-3-oxopropoxy)benzimidamido)azetidine-1-carboxylate (3_4_8)

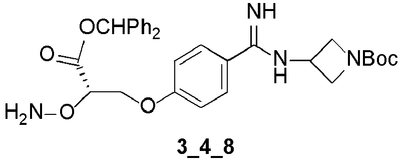

¹H NMR (400 MHz, DMSO-*d*₆): *δ* 1.39 (s, 9H), 3.41-3.46 (m, 3 H), 3.85-3.92 (m, 2H), 4.18-4.26 (m, 1 H), 4.34-4.38 (m, 2H), 4.01-4.45 (m, 1H), 4.61-4.64 (m, 1 H), 6.44 (s, 2 H), 6.90 (s, 1 H), 7.04 (d, *J* = 8.8 Hz, 2 H), 7.22-7.31 (m, 4H), 7.33-7.40 (m, 4H), 7.41-7.45 (m,2 H), 7.75 (d, *J* =8.8 Hz, 2 H). LC-MS analysis: [M+H]⁺ = 561.2.

### 3.5 tert-Butyl (R)-3-(4-((S)-2-(aminooxy)-3-(benzhydryloxy)-3-oxopropoxy)benzimidamido)pyrrolidine-1-carboxylate (3_5_8)

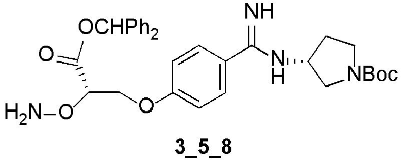

¹H NMR (400 MHz, DMSO-*d*₆): *δ* 1.34 (s, 9 H), 1.80-1.99 (m, 1 H), 2.11-2.18 (m, 1 H), 3.33-3.39 (m, 3 H), 3.52-3.61 (m, 1H), 4.27-4.39 (m, 3 H), 4.54-4.60 (m, 1 H), 6.38 (s, 2 H), 6.82 (s, 1 H), 7.02 (d, *J* = 7.8 Hz, 2 H), 7.17-7.24 (m, 4 H), 7.26-7.33 (m, 4 H), 7.34-7.38 (m, 2 H), 7.63 (d, *J* = 7.8 Hz, 2 H). LC-MS analysis: [M+H]⁺ = 575.3.

### 3.6 tert-Butyl (S)-3-(4-((S)-2-(aminooxy)-3-(benzhydryloxy)-3-oxopropoxy)benzimidamido)pyrrolidine-1-carboxylate (3_6_8)

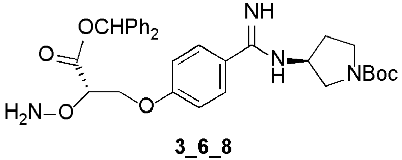

¹H NMR (400 MHz, DMSO-*d*₆): *δ* 1.41 (s, 9 H), 1.97-2.07 (m, 1 H), 2.17-2.27 (m, 1 H), 3.40-3.47 (m, 3 H), 3.60-3.68 (m, 1 H), 4.35-4.47 (m, 3 H), 4.62-4.65 (m, 1 H), 6.45 (s, 2 H), 6.90 (s, 1 H), 7.07 (d, *J* = 8.8 Hz, 2 H), 7.22-7.32 (m, 4 H), 7.33-7.40 (m, 4 H), 7.41-7.46 (m, 2 H), 7.73 (d, *J* = 8.8 Hz, 2 H). LC-MS analysis: [M+H]⁺ = 575.3.

### 4. Synthesis of the final compound

### Example 1

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-(piperidin-4-yl)carbamimidoyl)phenoxy)propanoic acid

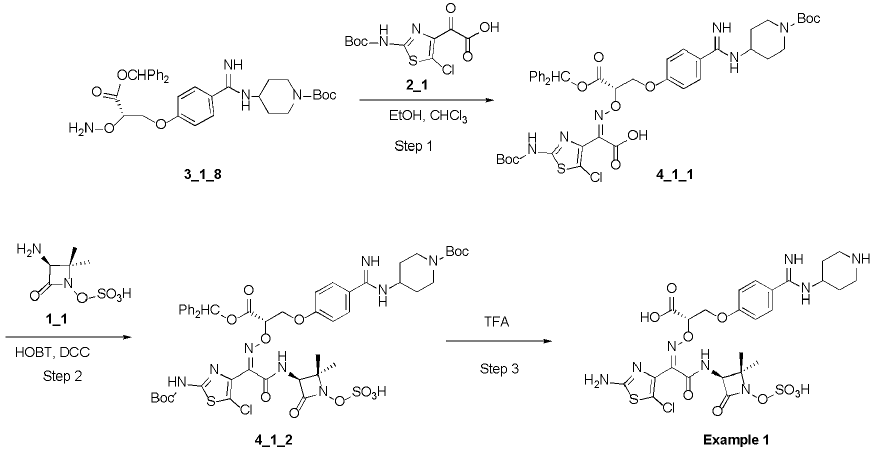

### Step 1: (S,Z)-2-(((1-(benzhydryloxy)-3-(4-(N-(1-(tert-butoxycarbonyl)piperidin-4-yl)carbamimidoyl)phenoxy)-1-oxopropan-2-yl)oxy)imino)-2-(2-((tert-butoxycarbonyl)amino)-5-chlorothiazol-4-yl)acetic acid (4_1_1)

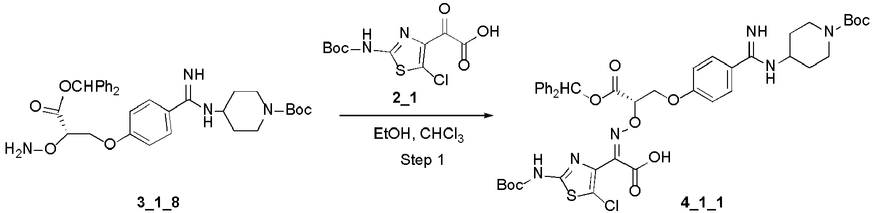

To the solution of compound **3_1_8** (0.294 g, 0.50 mmol) in ethanol (3 mL) and chloroform (3 mL) was added 2-(2-((*tert*-butoxycarbonyl)amino)-5-chlorothiazol-4-yl)-2-oxoacetic acid **2_1** (0.151 g, 0.490 mmol). The resulting mixture was stirred at room temperature for 18 hours, concentrated under reduced pressure, and purified by flash column chromatography (10-20% MeOH in CH₂Cl₂) to give compound **4_1_1** (0.31 g, 71% yield) as a pale yellow solid.

¹H NMR (400 MHz, DMSO-*d*₆): *δ* 1.42 (s, 9 H), 1.46 (s, 9 H), 1.54-1.43(m, 2 H), 1.86-1.96 (m, 2 H), 2.71-2.91 (m, 2 H), 3.84 -4.03 (m, 3 H), 4.43-4.57 (m, 2 H), 5.05-5.12 (m,1 H), 6.87 (s, 1 H), 7.09 (d, *J* = 8.8 Hz, 2 H), 7.20-7.30 (m, 6 H), 7.41-7.50 (m, 4 H), 7.68 (d, *J* = 8.8 Hz, 2 H), 9.20 (s, 1 H), 9.38 (s, 1 H), 9.47 (s, 1 H), 11.9 (s, 1 H). LC-MS analysis: [M+H]⁺ = 877.1 and [M+H+2]⁺= 879.1.

### Step 2: tert-Butyl 4-(4-((S)-3-(benzhydryloxy)-2-((((Z)-1-(2-((tert-butoxycarbonyl)amino)-5-chlorothiazol-4-yl)-2-(((S)-2,2-dimethyl-4-oxo-1-(sulfooxy)azetidin-3-yl)amino)-2-oxoethylidene)amino)oxy)-3-oxopropoxy)benzimidamido)piperidine-1-carboxylate (4_1_2)

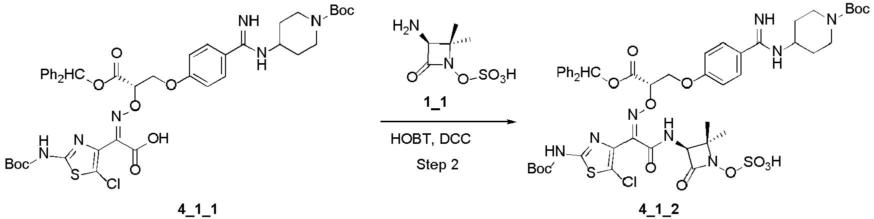

To the solution of compound **4_1_1** (0.305 g, 0.350 mmol) in anhydrous DMF (3.5 mL) was added DCC (0.11 g, 0.52 mmol) and HOBt (70.0 mg, 0.52 mmol). The resulting mixture was stirred at room temperature for 45 minutes, and then (*S*)-3-amino-2,2-dimethyl-4-oxoazetidin-1-ylhydrogen sulfate **1_1** (0.11 g, 0.52 mmol) and NaHCO₃ (88.0 mg, 1.05 mmol) were added, and continued to stir at room temperature overnight. The reaction mixture was concentrated under reduced pressure to give residue. The residue was purified by flash chromatography on silica gel eluting by 5-10% MeOH in CH₂Cl₂ to give compound **4_1_2** (0.32 g, 86% yield) as a pale yellow solid.

¹H NMR (400 MHz, DMSO-*d*₆): *δ* 1.11 (s, 3 H), 1.37 (s, 3 H), 1.41 (s, 9 H), 1.46 (s, 9 H), 1.44-1.51 (m, 2 H), 1.87-1.97 (m, 2 H), 2.72-2.89 (m, 2 H), 3.78-3.88 (m, 1 H), 3.94-4.04 (m, 2H), 4.45-4.52 (m, 1 H), 4.57 (d, *J* = 7.8 Hz, 1 H), 4.58-4.65 (m, 1 H), 5.33-5.38 (m,1 H), 6.92 (s, 1 H), 7.07 (d, *J* = 8.8 Hz, 2 H), 7.17-7.50 (m, 10 H), 7.70 (d, *J* = 8.8 Hz, 2 H), 8.98 (s, 1 H), 9.30 (s, 1 H), 9.38 (d, *J* = 7.8 Hz, 1 H), 9.63 (d, *J* = 7.8 Hz, 1 H), 12.1 (s, 1 H). LC-MS analysis: [M-H]⁻ =1066.8 and [M +2-H]⁻ =1068.8.

### Step 3: (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-(piperidin-4-yl)carbamimidoyl)phenoxy)propanoic acid (Example 1)

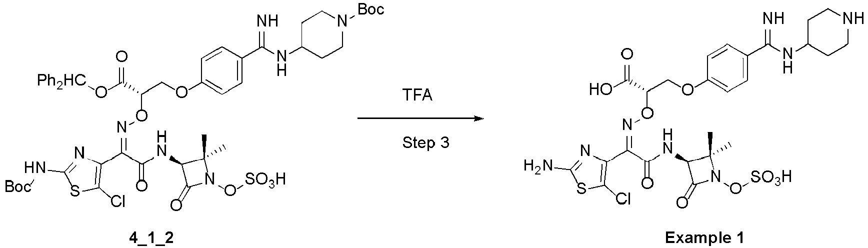

To the solution of compound **4_1_2** (0.32 g, 0.30 mmol) in anhydrous DCM (4 mL) was added TFA (3 mL) at 0 °C. After stirring for 1 hour at 0 °C, the resulting mixture was warmed to room temperature and stirred for additional 2 hours. The reaction mixture was concentrated under reduced pressure to give a residue. The residue was dissolved with water (20 mL) and washed with petroleum ether/EtOAc (2:1, 40 mL). The aqueous layer was freeze-dried to give the crude title compound (0.28 g) as a pale yellow power, which was purified by prep. HPLC on a Agilent 10 prep-C18 250x21.2 mm column and lyophilized to give the target Example **1** (16 mg, 7.6% yield) as a white solid.

¹H NMR (400 MHz, DMSO-*d*₆): *δ* 1.29 (s, 3 H), 1.42 (s, 3 H), 1.71-1.87 (m, 2 H), 1.98-2.12 (m, 2 H), 2.81-2.96 (m, 3 H), 3.80-3.91 (m, 2 H), 4.37-4.53 (m, 2 H), 4.69 (d, *J* = 8.8 Hz, 1 H), 4.73 (d, *J* = 7.8 Hz, 1 H), 7.20 (d, *J* = 8.2 Hz, 2 H), 7.36 (br s, 2 H), 7.69 (d, *J* = 8.2 Hz, 2 H), 8.99 (br s, 1 H), 9.45 (br s 1 H), 9.63 (br s, 1 H), 11.6 (d, *J* = 6.84 Hz, 1 H). LC-MS analysis: [M-H]⁻ =701.1 and [M +2-H]⁻ =703.2.

Following the procedure detailed above under example **1** but using the diffrent amines (amine **3_2_8** to **3_6_8),** the diffrent kitoacid **(2_1** to **2_2)** and the diffrent 3-amino-azetidin-2-one **(1_1** to **1_2),** the final compounds (example **2** to example **12** ) were prepared. For the convenience of the reader the structures of the final compounds as well as used building blocks (BB 1: **1_x,** BB2: **2_x** and BB3: **3**_**x_8**) are provided in table 1, which is followed by analytical data for all examples exept for example **1.**

Compounds of examples 2, 4, 6, 7 and 12 do not form part of the invention.

**Table 1. Structures of final compounds and used building blocks**

| **Example** | **Structure** | **BB 1 (1_x)** | **BB 2 (2_x)** | **BB 3 (3_x_8)** |
|---|---|---|---|---|
| 1 | 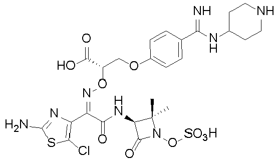 | 1_1 | 2_1 | 3_1_8 |
| **2** | 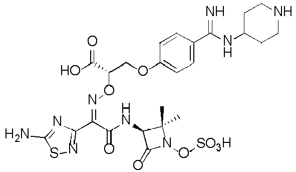 | 1_1 | 2_2 | 3_1_8 |
| 3 | 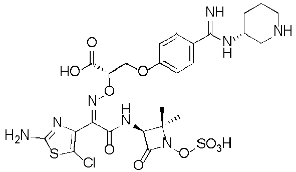 | 1_1 | 2_1 | 3_2_8 |
| **4** | 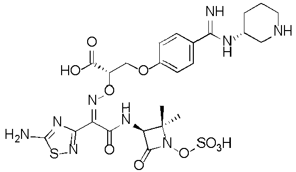 | 1_1 | 2_2 | 3_2_8 |
| **5** | 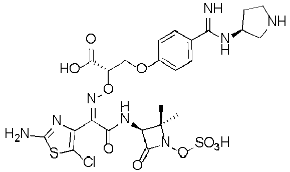 | 1_1 | 2_1 | 3_6_8 |
| **6** | 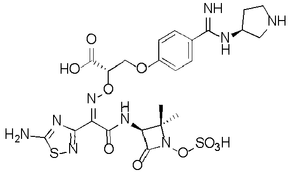 | 1_1 | 2_2 | 3_6_8 |
| **7** | 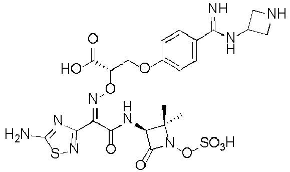 | 1_1 | 2_2 | 3_4_8 |
| 8 | 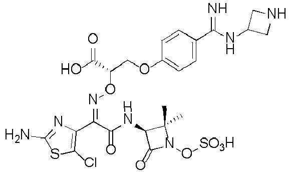 | 1_1 | 2_1 | 3_4_8 |
| **9** | 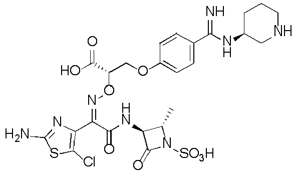 | 1_2 | 2_1 | 3_3_8 |
| **10** | 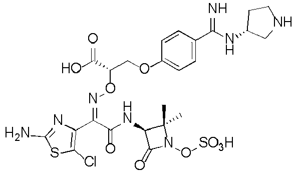 | 1_1 | 2_1 | 3_5_8 |
| **11** | 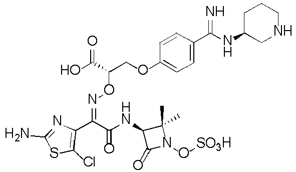 | 1_1 | 2_1 | 3_3_8 |
| **12** | 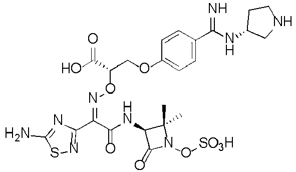 | 1_1 | 2_2 | 3_5_8 |

### Example 2

### (S)-2-((((Z)-1-(5-Amino-1,2,4-thiadiazol-3-yl)-2-(((S)-2,2-dimethyl-4-oxo-1-(sulfooxy)azetidin-3-yl)amino)-2-oxoethylidene)amino)oxy)-3-(4-(N-(piperidin-4-yl)carbamimidoyl)phenoxy)propanoic acid

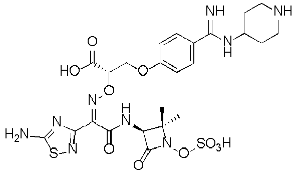

¹H NMR (400 MHz, DMSO-*d₆*): *δ* 1.23 (s, 3 H), 1.41 (s, 3 H), 1.71-1.85 (m, 2 H), 2.01-2.09 (m, 2 H), 2.81-2.93 (m, 3 H), 3.79-3.88 (m, 2 H), 4.36-4.50 (m, 2 H), 4.67 (d, *J* = 8.4 Hz, 1 H), 4.76 (d, *J* = 6.9 Hz, 1 H), 7.21 (d, *J* = 7.1 Hz, 2 H), 7.70 (d, *J* = 7.1 Hz, 2 H), 8.14 (br s, 1 H), 8.17 (br s, 1 H), 8.99 (br s, 1 H), 9.43 (br s, 1 H), 9.67 (br s, 1 H), 11.02 (br s, 1 H). LC-MS analysis: [M-H]⁻ = 667.9.

### Example 3

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((R)-piperidin-3-yl)carbamimidoyl)phenoxy)propanoic acid

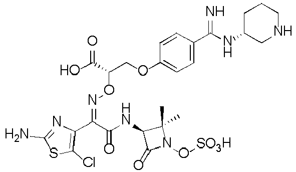

¹H NMR (400 MHz, DMSO-*d₆*): *δ* 1.27 (s, 3 H), 1.41 (s, 3 H), 1.62-1.74 (m, 2 H), 1.87-2.02 (m, 2 H), 2.82-2.92 (m, 2 H), 3.04-3.12 (m, 2 H), 3.82-3.93 (m, 1 H), 4.18-4.26 (m, 1 H), 4.39-4.46 (m, 1 H), 4.69 (d, *J* = 8.7 Hz, 1 H), 4.74 (d, *J* = 5.5 Hz, 1 H), 7.08 (d, *J* = 8.2 Hz, 2 H), 7.36 (br s, 2 H), 7.62 (d, *J* = 8.2 Hz, 2 H), 9.29 (br s, 2 H), 10.63 (br s 1 H). LC-MS analysis: [M-H]⁻ =700.9 and [M +2-H]⁻ =702.8.

### Example 4

### (S)-2-((((Z)-1-(5-Amino-1,2,4-thiadiazol-3-yl)-2-(((S)-2,2-dimethyl-4-oxo-1-(sulfooxy)azetidin-3-yl)amino)-2-oxoethylidene)amino)oxy)-3-(4-(N-((R)-piperidin-3-yl)carbamimidoyl)phenoxy)propanoic acid

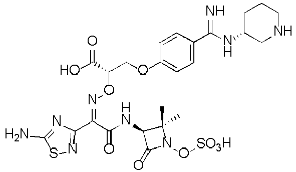

¹H NMR (400 MHz, DMSO-*d*₆): *δ* 1.21 (s, 3 H), 1.40 (s, 3 H), 1.69-1.79 (m, 2 H), 1.94-2.04 (m, 2 H), 2.87-3.01 (m, 2 H), 3.11-3.19 (m, 2 H), 3.84-3.92 (m, 1 H), 4.16-4.23 (m, 1 H), 4.41-4.47 (m, 1 H), 4.66 (d, *J* = 8.1 Hz, 1 H), 4.78-4.82 (m, 1 H), 7.02 (d, *J* = 8.6 Hz, 2 H), 7.59 (d, *J* = 8.6 Hz, 2 H), 8.14 (br s, 2 H), 9.02 (br s, 1 H), 9.23 (br s, 1 H), 11.04 (br s, 1 H). LC-MS analysis: [M-H]⁻ = 667.9.

### Example 5

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((S)-pyrrolidin-3-yl)carbamimidoyl)phenoxy)propanoic acid

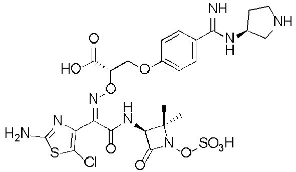

¹H NMR (400 MHz, DMSO-*d₆*): *δ* 1.27 (s, 3 H), 1.42 (s, 3 H), 2.14-2.27 (m, 2 H), 3.25-3.45 (m, 3 H), 3.57-3.64 (m, 1 H), 4.16 (d, *J* = 9.7 Hz, 1 H), 4.40 (t, *J* = 9.7 Hz, 1 H), 4.45-3.51 (m, 1 H), 4.63 (d, *J* = 8.4 Hz, 1 H), 4.77 (d, *J* = 8.7 Hz, 1 H), 7.09 (d, *J* = 8.3 Hz, 2 H), 7.36 (br s, 2 H), 7.75 (d, *J* = 8.3 Hz, 2 H), 9.04 (br s, 2 H), 10.35 (br s, 1 H). LC-MS analysis: [M-H]⁻ = 686.9 and [M +2-H]⁻=688.9.

### Example 6

### (S)-2-((((Z)-1-(5-Amino-1,2,4-thiadiazol-3-yl)-2-(((S)-2,2-dimethyl-4-oxo-1-(sulfooxy)azetidin-3-yl)amino)-2-oxoethylidene)amino)oxy)-3-(4-(N-((S)-pyrrolidin-3-yl)carbamimidoyl)phenoxy)propanoic acid

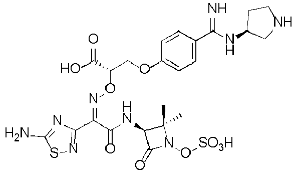

¹H NMR (400 MHz, DMSO-*d₆*): *δ* 1.19 (s, 3 H), 1.40 (s, 3 H), 2.04-2.23 (m, 2 H), 3.37-3.42 (m, 3 H), 3.57-3.63 (m, 1 H), 4.19-4.26 (m, 1 H), 4.30-4.44 (m, 2H), 4.63 (d, *J* = 8.4 Hz, 1 H), 4.76 (d, *J* = 7.8 Hz, 1 H), 7.11 (d, *J* = 7.8 Hz, 2 H), 7.74 (d, *J=* 7.8 Hz, 2 H), 8.23 (br s, 2 H), 10.35 (d, *J=* 8.4 Hz, 1 H). LC-MS analysis: [M-H]⁻ = 654.0.

### Example 7

### (S)-2-((((Z)-1-(5-Amino-1,2,4-thiadiazol-3-yl)-2-(((S)-2,2-dimethyl-4-oxo-1-(sulfooxy)azetidin-3-yl)amino)-2-oxoethylidene)amino)oxy)-3-(4-(N-(azetidin-3-yl)carbamimidoyl)phenoxy)propanoic acid

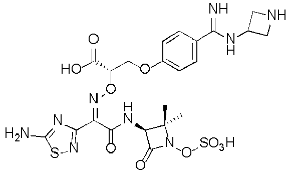

¹H NMR (400 MHz, DMSO-*d₆*): *δ* 1.15 (s, 3 H), 1.37 (s, 3 H), 4.01-4.09 (m, 2 H), 4.16-4.26 (m, 2 H), 4.29-4.39 (m, 2 H), 4.55-4.64 (m, 2 H), 4.78-4.86 (m, 1 H), 7.04 (d, *J* = 8.6 Hz, 2 H), 7.77 (d, *J* = 8.6 Hz, 2 H), 8.14 (br s, 2 H), 10.20 (m, 1 H). LC-MS analysis: [M-H]⁻ = 640.7.

### Example 8

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-(azetidin-3-yl)carbamimidoyl)phenoxy)propanoic acid

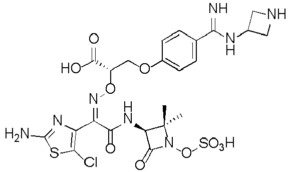

¹H NMR (400 MHz, DMSO-*d₆*): *δ* 1.29 (s, 3 H), 1.42 (s, 3 H), 4.12-4.17 (m, 1 H), 4.19-4.30 (m, 2 H), 4.34-4.41 (m, 2 H), 4.43-4.47 (m, 1 H), 4.61 (d, *J* = 8.3 Hz, 1 H), 4.63-4.65 (m, 1 H), 4.78-4.85 (m, 1 H), 7.21 (d, *J* = 8.8 Hz, 2 H), 7.33 (br s, 2 H), 7.69 (d, *J* = 8.8 Hz, 2 H), 8.38 (br s, 4 H), 11.6 (d, *J* = 8.3 Hz, 1 H). LC-MS analysis: [M-H]⁻ = 673.1 and [M +2-H]⁻=675.1.

### Example 9

### (S)-2-((((Z)-1-(2-Amino-5-chlorothiazol-4-yl)-2-(((2S,3S)-2-methyl-4-oxo-1-sulfoazetidin-3-yl)amino)-2-oxoethylidene)amino)oxy)-3-(4-(N-((S)-piperidin-3-yl)carbamimidoyl)phenoxy)propanoic acid

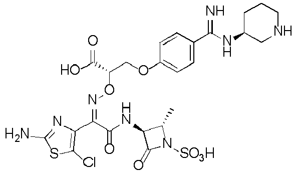

¹H NMR (400 MHz, DMSO-*d₆*): *δ* 1.35 (d, *J* = 6.4 Hz, 3 H), 1.42-1.49 (m, 1 H), 1.63-1.71 (m, 1 H), 1.79-1.87 (m, 1 H), 1.95-2.02 (m, 1 H), 2.86-2.95 (m, 1 H), 2.98-3.08 (m, 3 H), 3.60-3.65 (m, 1 H), 3.91-3.97 (m, 1 H), 4.13-4.19 (m, 1 H), 4.30 (t, *J* = 8.6 Hz, 1 H), 4.42 (dd, *J* = 7.9, 2.6 Hz, 1 H), 4.72 (d, *J* = 7.4 Hz, 1 H), 6.65 (br s, 1 H), 7.14 (d, *J* = 8.7 Hz, 2 H), 7.19 (br s, 2 H), 7.72 (d, *J* = 8.7 Hz, 2 H). LC-MS analysis: [M-H]⁻ = 671.1 and [M+2-H]⁻ =673.1.

### Example 10

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((R)-pyrrolidin-3-yl)carbamimidoyl)phenoxy)propanoic acid

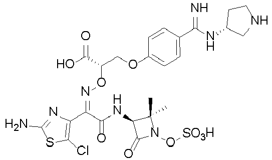

¹H NMR (400 MHz, DMSO-*d₆*): *δ* 1.27 (s, 3 H), 1.41 (s, 3 H), 2.00-2.09 (m, 1 H), 2.17-2.26 (m, 1 H), 3.07-3.15 (m, 1 H), 3.21-3.36 (m, 3 H), 4.22-4.27 (m, 1 H), 4.36-4.44 (m, 2 H), 4.65 (t, *J* = 8.6 Hz, 1 H), 4.72 (d, *J* = 8.5 Hz, 1 H), 7.13 (d, *J* = 8.5 Hz, 2 H), 7.35 (br s, 2 H), 7.74 (d, *J* = 8.5 Hz, 2 H), 8.30 (br s, 3 H), 10.35 (d, *J* = 8.6 Hz, 1 H). LC-MS analysis: [M-H]⁻ = 687.1 and [M-H+2]⁻ = 689.1.

### Example 11

### (S)-2-((((Z)-1-(2-Amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((S)-piperidin-3-yl)carbamimidoyl)phenoxy)propanoic acid

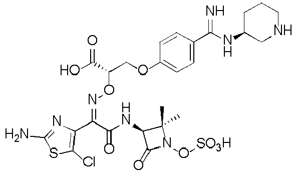

¹H NMR (400 MHz, DMSO-*d₆*): *δ* 1.26 (s, 3 H), 1.41 (s, 3 H), 1.54-1.79 (m, 3 H), 1.92-2.02 (m, 1 H), 2.73-2.81 (m, 1 H), 2.87-2.98 (m, 3 H), 3.84-3.91 (m, 1 H), 4.10-4.16 (m, 1 H), 4.35 (t, *J* = 9.5 Hz, 1 H), 4.67 (d, *J* = 8.8 Hz, 1 H), 4.70 (d, *J* = 8.5 Hz, 1 H), 7.13 (d, *J* = 8.5 Hz, 2 H), 7.33 (br s, 2 H), 7.72 (d, *J* = 8.5 Hz, 2 H), 9.21 (br s, 3 H), 11.08 (br s, 1 H). LC-MS analysis: [M-H]⁻ = 701.1 and [M+2-H]⁻ = 703.1.

### Example 12

### (S)-2-((((Z)-1-(5-Amino-1,2,4-thiadiazol-3-yl)-2-(((S)-2,2-dimethyl-4-oxo-1-(sulfooxy)azetidin-3-yl)amino)-2-oxoethylidene)amino)oxy)-3-(4-(N((R)-pyrrolidin-3-yl)carbamimidoyl)phenoxy)propanoic acid

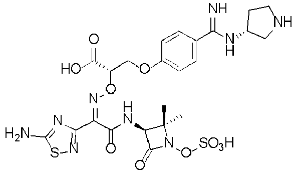

¹H NMR (400 MHz, DMSO-*d*₆): *δ* 1.26 (s, 3 H), 1.43 (s, 3 H), 1.98-2.03 (m, 1 H), 2.07-2.15 (m, 1 H), 3.27-3.34 (m, 1 H), 3.35-3.45 (m, 2 H), 3.46-3.54 (m, 1 H), 4.16-4.21 (m, 1 H), 4.40-4.45 (m, 2 H), 4.69 (d, *J* = 8.7 Hz, 1 H), 4.72 (t, *J* = 4.9 Hz, 1 H), 7.18 (d, *J* = 8.7 Hz, 2 H), 7.72 (d, *J* = 8.7 Hz, 2 H), 8.12 (br s, 2 H), 8.41 (br s, 5 H), 11.25 (br s, 1 H). LC-MS analysis: [M-H]⁻ = 654.2.

### Biological Assay

The antimicrobial activity of the compounds of this invention against a selection of different bacteria may be evaluated by a number of assays, including the *in-vitro* determination of the minimum inhibitory concentration (MIC) or the determination of the *in-vivo* efficacy in mouse infection models.

### Minimum Inhibitory Concentration (MIC) Determination

Compounds of this invention were tested for antimicrobial activity by determining minimum inhibitory concentrations (MICs, in µg/mL) using the broth microdilution method according to the guidelines of the Clinical Laboratories and Standards Institute ("Methods for Dilution Antimicrobial Susceptibility Tests for Bacteria that Grow Aerobically", Approved standard, 7th ed., Clinical and Laboratory Standards Institute (CLSI) Document M7-A8, Wayne, Pa., USA, 2009.). Test compounds were dissolved in DMSO. The compounds were then diluted in microbial growth medium (Mueller Hinton Broth II, cation adjusted) resulting in a final concentration range of 0.063-64 µg/mL in serial two-fold dilution. In all cases the final DMSO concentration was less than 0.5%. Bacteria were added to 96-well microtitre plates containing the two-fold dilutions of the compounds; the final cell density was approximately 5× 10⁵ colony forming units/mL (CFU/mL). Plates were incubated at 37°C for 18-24 hours and read visually. The MIC, i.e. the lowest concentration of the test compound that inhibited visible growth of the bacteria, was recorded. The same assay conditions were used when the compounds of this invention were tested in combination with β-lactamase inhibitors. While the compounds of this invention were serially diluted as described above, a constant concentration of the inhibitor of 4 mg/L was used.

Bacterial strains that were used to evaluate the antimicrobial acitivity using the MIC determination included but were not limited to *E. coli* clinical isolate, *E. coli* 8739, *K. pneumoniae* clinical isolate, *K. pneumoniae* 700603, *E. cloacae* clinical isolate, *E. cloacae* 700323, *A. baumannii* clinical isolate, *A. baumannii* 19606, *P. aeruginosa* clinical isolate, *P. aeruginosa* 9027.

**Table 1. Biological data**

| compound example No. | Strain 1 MIC [mg/L] | Strain 2 MIC [mg/L] | Strain 3 MIC [mg/L] | Strain 4 MIC [mg/L] | Strain5 MIC [mg/L] |
|---|---|---|---|---|---|
| AZT | >64 | 16 | 64 | 64 | >64 |
| CAZ | >64 | >64 | >64 | >64 | >64 |
| 1 | 8 | 1 | 4 | 8 | 8 |
| 2 | 0.5 | 0.125 | 16 | 64 | 8 |
| 3 | 2 | 0.5 | 2 | 8 | 8 |
| 4 | 0.25 | 0.25 | 8 | 64 | 16 |
| 5 | 0.25 | 0.25 | 1 | 4 | 2 |
| 6 | 0.5 | 0.125 | 1 | 64 | 4 |
| 7 | 0.125 | 0.25 | 2 | 32 | 8 |
| 8 | 0.5 | 0.25 | 8 | 8 | 8 |
| 9 | 1 | 0.5 | 32 | 64 | 64 |
| 10 | 1 | 0.25 | 1 | 1 | 2 |
| 11 | 0.5 | 0.5 | 32 | 64 | 32 |
| 12 | 8 | 1 | 4 | 8 | 8 |

| compound example No. | Strain 6 MIC [mg/L] | Strain 7 MIC [mg/L] | Strains MIC [mg/L] | Strain 9 MIC [mg/L] | Strain 10 MIC [mg/L] |
|---|---|---|---|---|---|
| AZT | >64 | >64 | 32 | >64 | 16 |
| CAZ | >64 | >64 | >64 | >64 | >64 |
| 1 | 8 | 16 | 8 | 2 | 2 |
| 2 | 64 | 8 | 32 | 1 | 1 |
| 3 | 8 | 16 | 8 | 4 | 1 |
| 4 | 64 | 8 | 16 | 2 | 1 |
| 5 | 4 | 8 | 4 | 1 | 0.5 |
| 6 | 32 | 4 | 16 | 2 | 0.5 |
| 7 | 16 | 4 | 8 | 0.5 | 0.5 |
| 8 | 8 | 8 | 4 | 1 | 1 |
| 9 | 32 | 32 | 16 | 16 | 1 |
| 10 | 4 | 8 | 8 | 2 | 2 |
| 11 | 64 | 16 | 32 | 4 | 2 |
| 12 | 8 | 16 | 8 | 2 | 2 |

| | | | | | |
|---|---|---|---|---|---|
| AZT: Aztreonam; CAZ: Ceftazidime | | | | | |

| | |
|---|---|
| strain 1 | E. coli clinical isolate |
| strain 2 | E. coli 8739 |
| strain 3 | K. pneumoniae clinical isolate |
| strain 4 | K. pneumoniae 700603 |
| strain 5 | E. cloacae clinical isolate |
| strain 6 | E. cloacae 700323 |
| strain 7 | A. baumannii clinical isolate |
| strain 8 | A. baumannii 19606 |
| strain 9 | P. aeruginosa clinical isolate |
| strain 10 | P. aeruginosa 9027 |

## Claims

1. A compound of formula (I) 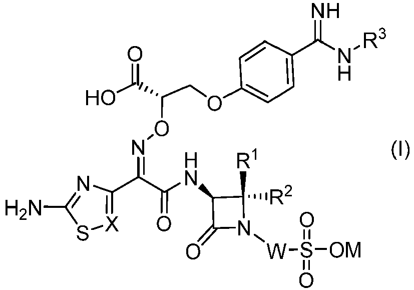 in which
M is hydrogen or a pharmaceutically acceptable salt forming cation,
R¹ and R² represent independently of one another hydrogen or methyl,
W represents a bond or O,
X represents CR⁴,
R⁴ represents Cl,
R³ represents hydrogen, amino, hydroxy, (C₁-C₆)-alkyl or a 4-, 5- or 6-membered nitrogen-containing heterocycle, whereby alkyl may be substituted with a substituent selected from the group consisting of hydroxy, amino, carboxy, carbonyloxy, mono- or di-(C₁-C₆)-alkylamino, -NH-CH(=NH), -NH-C(=NH)(NH₂), 5- or 6-membered nitrogen-containing heteroaryl and 5- or 6-membered nitrogen-containing heterocyclyl, and the salts thereof, the solvates thereof and the solvates of the salts thereof, or a deuterated compound of any such compound.

2. Compound according to claim 1, **characterized in that**
M is hydrogen or a pharmaceutically acceptable salt forming cation,
R¹ and R² represent methyl,
W represents O,
X represents CR⁴
R⁴ represents Cl,
R³ represents aminoethyl, azetidine, pyrrolidine or piperidine, and the salts thereof, the solvates thereof and the solvates of the salts thereof, or a deuterated compound of any such compound.

3. Compound according to any one of claims 1 to 2, which is selected from the following group of compounds: 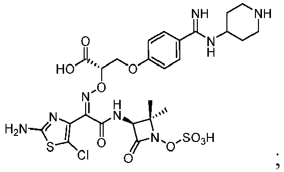 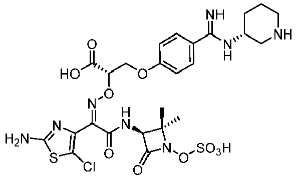 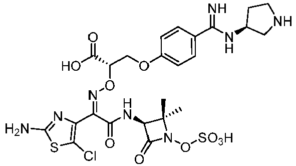 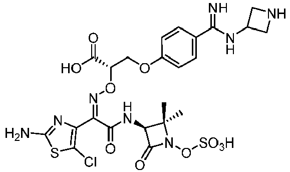 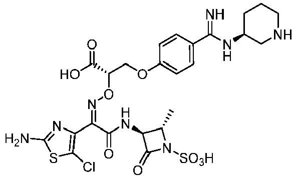 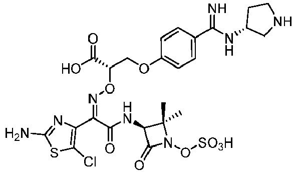 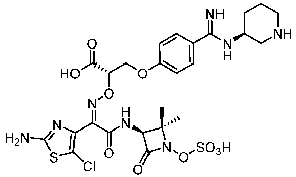 and pharmaceutically acceptable salts of such compounds, and deuterated compounds of such compounds and salts.

4. The compound of formula (I) as defined in claim 1, wherein the compound is selected from the group consisting of:
(S)-2-((((Z)-1-(2-amino-5-chlorothiazol-4-yl)-2-(((S)-2,2-dimethyl-4-oxo-1-sulfoazetidin-3-yl)amino)-2-oxoethylidene)amino)oxy)-3-(4-(N-(2-aminoethyl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-(2-aminoethyl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-(piperidin-4-yl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((R)-piperidin-3-yl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((S)-piperidin-3-yl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-(azetidin-3-yl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((R)-pyrrolidin-3-yl)carbamimidoyl)phenoxy)propanoic acid
(S)-2-((((Z)-1-(2-amino-5-chlorothiazol-4-yl)-2-(((S)-2,2-dimethyl-4-oxo-1-(sulfooxy)azetidin-3-yl)amino)-2-oxoethylidene)amino)oxy)-3-(4-(N-((S)-pyrrolidin-3-yl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-(piperidin-4-yl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-((R)-piperidin-3-yl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-((S)-piperidin-3-yl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-(azetidin-3-yl)carbamimidoyl)phenoxy)propanoic acid
(S)-2-((((Z)-1-(2-amino-5-chlorothiazol-4-yl)-2-(((2S,3S)-2-methyl-4-oxo-1-sulfoazetidin-3-yl)amino)-2-oxoethylidene)amino)oxy)-3-(4-(N-((R)-pyrrolidin-3-yl)carbamimidoyl)phenoxy)propanoic acid
(S)-2-((((Z)-1-(2-amino-5-chlorothiazol-4-yl)-2-(((25,3 S)-2-methyl-4-oxo-1-sulfoazetidin-3-yl)amino)-2-oxoethylidene)amino)oxy)-3-(4-(N-((S)-pyrrolidin-3-yl)carbamimidoyl)phenoxy)propanoic acid
or deuterated compound of any such compound.

5. The compound according to any one of claims 1 to 4 for use in method of treating and/or preventing diseases, in particular bacterial infections, especially with Gram-negative bacteria.

6. A medicament comprising at least one compound according to any one of claims 1 to 4 in combination with at least one further active compound.

7. The medicament according to claim 6, **characterized in that** the further active compound is a *β-*lactamase inhibitor, preferably selected from the group consisting of clavulanic acid, tazobactam, sulbactam, avibactam, relebactam, vaborbactam.

8. The medicament comprising at least one compound according to any one of claims 1 to 4 in combination with at least one inert, non-toxic, pharmaceutically acceptable excipient.

9. The medicament according to any one of claims 6 to 8 for use in method of treating and /or preventing bacterial infections.

10. A process for preparing the compound of claim 1, comprising a step of removing the protecting group from compounds of formula (II) 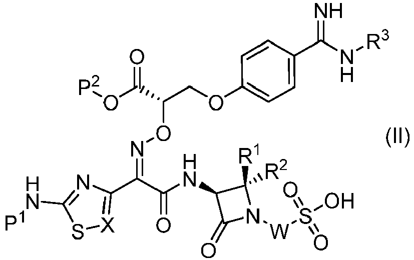 in which P¹ and P² represent independently a protecting group and R¹, R², R³, W and X are defined in claim 1, under acidic conditions.

## Patentansprüche

1. Eine Verbindung der Formel (I) 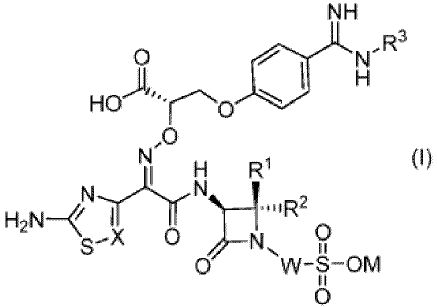 wobei
M ist Wasserstoff oder ein pharmazeutisch annehmbares Salz bildendes Kation,
R¹ und R² steht für unabhängig voneinander Wasserstoff oder Methyl,
W steht für eine Bindung oder O,
X steht für CR⁴,
R⁴ steht für Cl,
R³ steht für Wasserstoff, Amino, Hydroxy, (C₁-C₆)-Alkyl oder einen 4-, 5- oder 6-gliedrigen Stickstoff haltigen Heterozyklus darstellt,
wobei Alkyl mit einem Substituenten aus der Gruppe bestehend aus Hydroxy, Amino, Carboxy, Carbonyloxy, Mono- oder Di-(C₁-C₆)-Alkylamino, -NH-CH(=NH), -NH-C(=NH)(NH₂), 5- oder 6-gliedrigem Stickstoff haltigen Heteroaryl und 5- oder 6-gliedrigem Stickstoff haltigem Heterozyklyl substituiert sein kann,
und deren Salze, deren Solvate und die Solvate der Salze davon, oder eine deuterierte Verbindung einer dieser Verbindungen.

2. Verbindung nach Anspruch 1, **dadurch gekennzeichnet, dass**
M ist Wasserstoff oder ein pharmazeutisch annehmbares Salz bildendes Kation,
R¹ und R² steht für Methyl,
W steht für O,
X steht für CR⁴,
R⁴ steht für Cl,
R³ steht für Aminoethyl, Azetidin, Pyrrolidin oder Piperidin, und deren Salze, deren Solvate und die Solvate der Salze davon, oder eine deuterierte Verbindung einer dieser Verbindungen.

3. Verbindung nach einem der Ansprüche 1 bis 2, ausgewählt aus der folgenden Gruppe von Verbindungen: 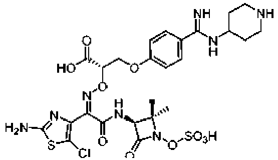 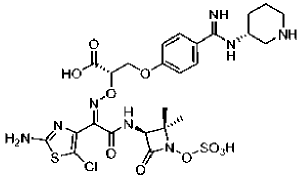 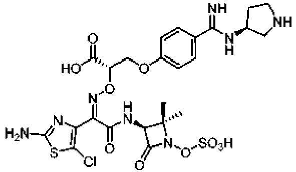 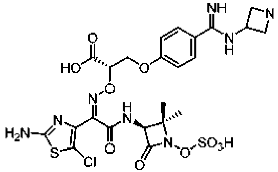 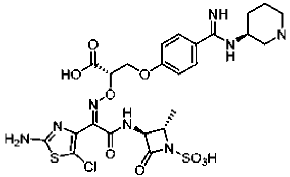 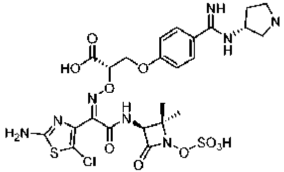 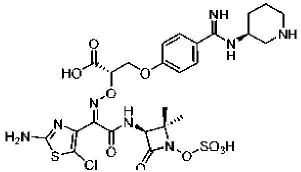 und pharmazeutisch annehmbare Salze dieser Verbindungen, und deuterierte Verbindungen dieser Verbindungen und Salze.

4. Verbindung der Formel (I) nach Anspruch 1, wobei die Verbindung ausgewählt ist aus der Gruppe bestehend aus:
(S)-2-((((Z)-l-(2-Amino-5-Chlorothiazol-4-yl)-2-(((S)-2,2-Dimethyl-4-Oxo-l-Sulfoazetidin-3-yl)Amino)-2-Oxoethyliden)Amino)Oxy)-3-(4-(N-(2-Aminoethyl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3-yl)Amino)-2-Oxoethyliden)Amino)Oxy)-3-(4-(N-(2-Aminoethyl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-(Piperidin-4-yl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-((R)-Piperidin-3-yl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-((S)-Piperidin-3-yl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-(Azetidin-3-yl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-((R)-Pyrrolidin-3-yl)Carbamimidoyl)Phenoxy)Propansäure
(S)-2-((((Z)-l-(2-Amino-5-Chlorothiazol-4-yl)-2-(((S)-2,2-Dimethyl-4-Oxo-l-(Sulfooxy)Azetidin-3-yl)Amino)-2-Oxoethyliden)Amino)Oxy)-3-(4-(N-((S)-Pyrrolidin-3-yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3- yl)Amino)-2-Oxoethyliden)Amino)Oxy)-3-(4-(N-(Piperidin-4-yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3- yl)Amino)-2-Oxoethyliden)Amino)Oxy)-3-(4-(N-((R)-Piperidin-3- yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3- yl)Amino)-2-Oxoethyliden)Amino)Oxy)-3-(4-(N-((S)-Piperidin-3-yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3- yl)Amino)-2-Oxoethyliden)Amino)Oxy)-3-(4-(N-(Azetidin-3-yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3-yl)Amino)-2-Oxoethyliden)Amino)Oxy)-3-(4-(N-((R)-Pyrrolidin-3-yl)Carbamimidoyl)Phenoxy)Propansäure
(S)-2-((((Z)-l-(2-Amino-5-Chlorothiazol-4-yl)-2-(((2S,3S)-2-Methyl-4-Oxo-l-Sulfoazetidin-3-yl)Amino)-2-Oxoethyliden)Amino)Oxy)-3-(4-(N-((S)-Pyrrolidin-3-yl)Carbamimidoyl)Phenoxy)Propansäure
oder deuterierte Verbindung einer dieser Verbindungen.

5. Verbindung nach einem der Ansprüche 1 bis 4 zur Verwendung in einem Verfahren zur Behandlung und/oder Vorbeugung von Krankheiten, insbesondere von bakteriellen Infektionen, insbesondere mit gramnegativen Bakterien.

6. Ein Medikament, umfassend mindestens eine Verbindung nach einem der Ansprüche 1 bis 4 in Kombination mit mindestens einer weiteren aktiven Verbindung.

7. Medikament nach Anspruch 6, **dadurch gekennzeichnet, dass** die weitere aktive Verbindung ein β-Laktamase-Inhibitor ist, vorzugsweise ausgewählt aus der Gruppe bestehend aus Clavulansäure, Tazobactam, Sulbactam, Avibactam, Relebactam, Vaborbactam.

8. Medikament, umfassend mindestens eine Verbindung nach einem der Ansprüche 1 bis 4 in Kombination mit mindestens einem inerten, nicht-toxischen, pharmazeutisch annehmbaren Hilfsstoff.

9. Medikament nach einem der Ansprüche 6 bis 8 zur Verwendung in einem Verfahren zur Behandlung und/oder Vorbeugung bakterieller Infektionen.

10. Ein Verfahren zur Herstellung der Verbindung nach Anspruch 1, umfassend einen Schritt der Entfernung der Schutzgruppe von Verbindungen der Formel (II) 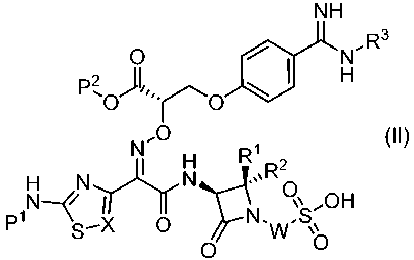 wobei P¹ und P² unabhängig voneinander eine Schutzgruppe darstellen und R¹, R², R³, W und X nach Anspruch 1 definiert sind, unter sauren Bedingungen.

## Revendications

1. Composé de Formule (I) 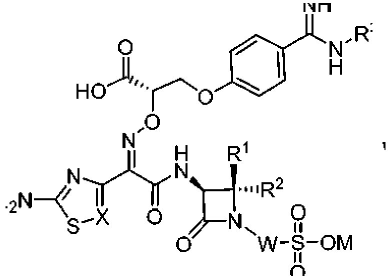 où,
M est l'hydrogène ou un cation formant un sel pharmaceutiquement acceptable,
R¹ et R² représentent indépendamment l'un de l'autre l'hydrogène ou le méthyle,
W représente une liaison ou O,
X représente CR⁴,
R⁴ représente Cl,
R³ représente l'hydrogène, l'amino, l'hydroxy, (Ci-C6)-alkyle ou l'hétérocycle azoté à 4, 5 ou 6 chaînons,
par lequel l'alkyle peut être substitué par un substituant choisi dans un groupe consistant en l'hydroxy, l'amino, le carboxy, le carbonyloxy, le mono- ou di-(Ci-C₆)-alkylamino, -NH-CH(=NH), -NH-C(=NH)(NH₂), l'hétéroaryle azoté à 5 ou 6 chaînons et l'hétérocyclyle azoté à 5 ou 6 chaînons,
et leurs sels, leurs solvates et les solvates de leurs sels, ou un composé deutéré de l'un des composés.

2. Composé selon la revendication 1, **caractérisé en ce que**,
M est l'hydrogène ou un cation formant un sel pharmaceutiquement acceptable,
R¹ et R² représentent le méthyle,
W représente O,
X représente CR⁴,
R⁴ représente Cl,
R³ représente l'aminoéthyle, l'azétidine, la pyrrolidine ou la pipéridine,
et leurs sels, leurs solvates et les solvates de leurs sels, ou un composé deutéré de l'un des composés.

3. Composé selon l'une quelconque des revendications 1 à 2, qui est choisi dans un groupe consistant en composés : 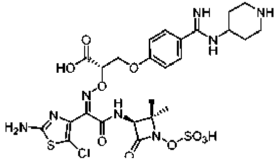 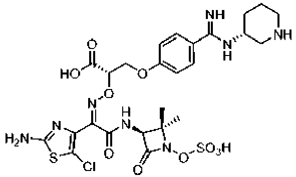 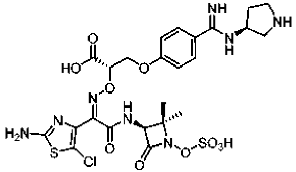 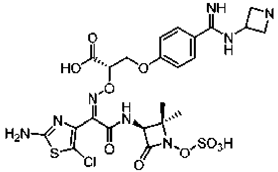 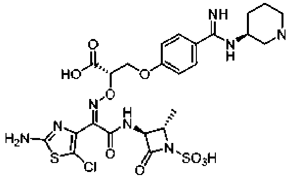 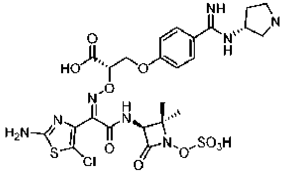 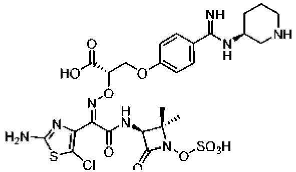 et des sels pharmaceutiquement acceptables des composés, et des composés deutérés des composés et des sels.

4. Composé de formule (I) selon la revendication 1, dans lequel le composé est choisi dans un groupe consistant en :
(S)-2-((((Z)-l-(2-amino-5-chlorothiazol-4-yl)-2-(((S)-2,2-diméthyl-4-oxo-l-sulfoa zétidine-3-yl)amino)-2-oxoéthylidène)amino)oxy)-3-(4-(N-(2-aminoéthyl)carbamimid oyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)-3-(4-(N-(2-aminoéthyl)carbamimidoyl) phénoxy)acide propanoïque
(S)-2-(((Z)-l-(2-amino-5-chlorothiazol-4-yl)-2-(((S)-2,2-diméthyl-4-oxo-l-(sulfo oxy)azétidine-3-yl)amino)-2-oxoéthylidène)amino)-3-(4-(N-(pipéridine-4-yl)carbami midoyl)phénoxy)acide propanoïque
(S)-2-((((Z)-l-(2-amino-5-chlorothiazol-4-yl)-2-((S)-2,2-diméthyl-4-oxo-l-(sulfo oxy)azétidine-3-yl)amino)-2-oxoéthylidène)amino)-3-(4-(N-((R)-pipéridine-3-yl)carb amimidoyl)phénoxy)acide propanoïque
(S)-2-((((Z)-l-(2-amino-5-chlorothiazol-4-yl)-2-(((S)-2,2-diméthyl-4-oxo-l-(sulfo oxy)azétidine-3-yl)amino)-2-oxoéthylidène)amino)-3-(4-(N-((S)-pipéridine-3-yl)carb amimidoyl)phénoxy)acide propanoïque
(S)-2-(((Z)-l-(2-amino-5-chlorothiazol-4-yl)-2-(((S)-2,2-diméthyl-4-oxo-l-(sulfo oxy)azétidine-3-yl)amino)-2-oxoéthylidène)amino)-3-(4-(N-(azétidine-3-yl)carbamim idoyl)phénoxy)acide propanoïque
(S)-2-((((Z)-l-(2-amino-5-chlorothiazol-4-yl)-2-(((S)-2,2-diméthyl-4-oxo-l-(sulfo oxy)azétidine-3-yl)amino)-2-oxoéthylidène)amino)-3-(4-(N-((R)-pyrrolidine-3-yl)car bamimidoyl)phénoxy)acide propanoïque
(S)-2-((((Z)-l-(2-amino-5-chlorothiazol-4-yl)-2-(((S)-2,2-diméthyl-4-oxo-l-(sulfo oxy)azétidine-3-yl)amino)-2-oxoéthylidène)amino)-3-(4-(N-((S)-pyrrolidine-3-yl)carb amimidoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-(pipéridine-4-yl)carbami midoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-((R)-pipéridine-3-yl)carb amimidoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-((S)-pipéridine-3-yl)carb amimidoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-(azétidine-3-yl)carbamim idoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-((R)-pyrrolidine-3-yl)car bamimidoyl)phénoxy)acide propanoïque
(S)-2-((((Z)-l-(2-amino-5-chlorothiazol-4-yl)-2-(((2S,3S)-2-méthyl-4-oxo-l-sulfo azétidine-3-yl)amino)-2-oxoéthylidène)amino)oxy)-3-(4-(N-((S)-pyrrolidine-3-yl)carb amimidoyl)phénoxy)acide propanoïque
ou composé deutéré d'un tel composé.

5. Composé selon l'une quelconque des revendications 1 à 4 pour l'utilisation dans un procédé de traitement et/ou de prévention de maladies, en particulier d'infections bactériennes, spécifique à bactéries à Gram négatif.

6. Médicament comprenant au moins un composé selon l'une quelconque des revendications 1 à 4 en combinaison avec au moins un composé actif supplémentaire.

7. Médicament selon la revendication 6, **caractérisé en ce que** le composé actif supplémentaire est un inhibiteur de -lactamase, de préférence choisi dans un groupe consistant en l'acide clavulanique, le tazobactam, le sulbactam, l'avibactam, le relebactam, et le vaborbactam.

8. Médicament comprenant au moins un composé selon l'une quelconque des revendications 1 à 4 en combinaison avec au moins un excipient inerte, non toxique et pharmaceutiquement acceptable.

9. Médicament selon l'une quelconque des revendications 6 à 8 pour l'utilisation dans un procédé de traitement et/ou de prévention d'infections bactériennes.

10. Procédé de préparation du composé selon la revendication 1, comprenant une étape d'éliminer le groupe protecteur des composés de formule (II) 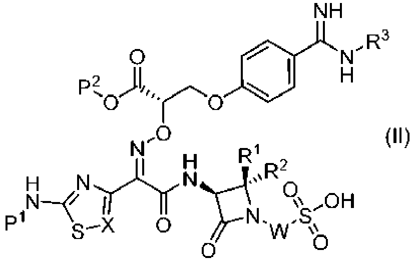 où P¹ et P² représentent indépendamment l'un de l'autre un groupe protecteur et R¹, R², R³, W et X sont définis dans la revendication 1, dans des conditions acides.
